(19) Europäisches Patentamt
European Patent Office
Office européen des brevets

(11) Veröffentlichungsnummer: **0 312 773 B1**

# (12) EUROPÄISCHE PATENTSCHRIFT

(45) Veröffentlichungstag der Patentschrift: **27.12.91**

(51) Int. Cl.5: **C07D 413/04**, C07D 417/04, A61K 31/445, A61K 31/495

(21) Anmeldenummer: **88115165.8**

(22) Anmeldetag: **16.09.88**

## (54) Substituierte 3-Aminosydnonimine, Verfahren zu ihrer Herstellung und ihre Verwendung.

(30) Priorität: **24.09.87 DE 3732174**

(43) Veröffentlichungstag der Anmeldung:
**26.04.89 Patentblatt 89/17**

(45) Bekanntmachung des Hinweises auf die Patenterteilung:
**27.12.91 Patentblatt 91/52**

(84) Benannte Vertragsstaaten:
**AT BE CH DE ES FR GB GR IT LI NL SE**

(56) Entgegenhaltungen:
**EP-A- 0 023 343**
**CH-A- 507 276**
**US-A- 3 769 283**

(73) Patentinhaber: **CASSELLA Aktiengesellschaft**
**Hanauer Landstrasse 526**
**W-6000 Frankfurt am Main 61(DE)**

(72) Erfinder: **Schönafinger, Karl, Dr.**
**Holunderweg 8**
**W-8755 Alzenau(DE)**
Erfinder: **Beyerle, Rudi, Dr.**
**Mitterfeld 5 d**
**W-8132 Tutzing(DE)**
Erfinder: **Bohn, Helmut, Dr.**
**Kranzbergring 11**
**W-6369 Schöneck 1(DE)**
Erfinder: **Just, Melitta, Dr.**
**Weimarer Strasse 2**
**W-6369 Niderau 2(DE)**

(74) Vertreter: **Urbach, Hans-Georg, Dr. et al**
**Hanauer Landstrasse 526**
**W-6000 Frankfurt am Main 61(DE)**

EP 0 312 773 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft pharmakologisch wirksame substituierte 3-Aminosydnonimine der allgemeinen Formel I

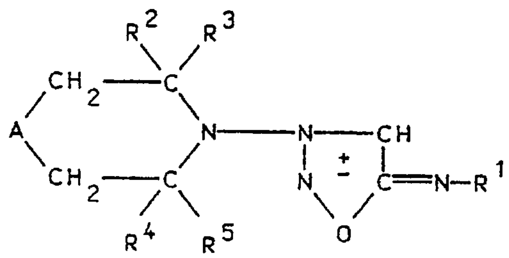

(I)

und ihre pharmakologisch annehmbaren Säureadditionssalze, worin
A den Rest $-CH_2-$, -O-, $-S(N)_n-$, $-N(R^6)-$ oder eine direkte Bindung;
$R^1$ Wasserstoff oder den Rest $-COR^7$;
$R^2$, $R^3$, $R^4$, $R^5$ Alkyl mit 1 bis 4 C-Atomen;
n die Zahl 0, 1 oder 2;
$R^6$ Alkyl mit 1 bis 4 C-Atomen; Hydroxyalkyl mit 1 bis 4 C-Atomen; Phenylalkyl mit 1 bis 4 C-Atomen im Alkylrest;
$R^7$ einen aliphatischen Rest mit 1 bis 4 C-Atomen, der auch durch Alkoxy mit 1 bis 3 C-Atomen substituiert sein kann; einen cycloaliphatischen Rest mit 5 bis 7 C-Atomen; einen bicycloaliphatischen Rest mit 5 bis 7 C-Atomen; einen tricycloaliphatischen Rest mit 7 bis 16 C-Atomen; einen Alkoxyrest mit 1 bis 6 C-Atomen; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen durch 1 bis 3 Halogenatome und/oder 1 bis 3 Alkylreste mit 1 bis 3 C-Atomen und/oder 1 bis 3 Alkoxyreste mit 1 bis 3 C-Atomen und/oder 1 oder 2 Nitrogruppen mono-, di- oder trisubstituierten Arylrest mit 6 bis 10 C-Atomen;
bedeuten.

Die Erfindung betrifft ferner ein Verfahren zur Herstellung der erfindungsgemäßen Verbindungen und ihre Verwenung.

Falls A einen der Reste $-CH_2-$, -O- $-S(O)_n-$, oder $-N(R^6)$-bedeutet, steht in 3-Stellung des Sydnonimins der Rest eines heterocyclischen 6-Rings mit einem Heteroatom (N) oder mit zwei Heteroatomen (N,O oder N,S oder N,N), der in der angegebenen Weise tetraalkyliert ist. Falls A eine direkte Bindung bedeutet, steht in 3-Stellung des Sydnonimins ein in 2,2,5,5-Stellung tetraalkylierter Pyrrolidinrest.

Für n ist die Zahl 2 bevorzugt.

Von den für A stehenden zweiwertigen Resten sind die Reste: $-CH_2-$, -O- und $-N(R^6)-$ bevorzugt.

Für $R^1$ ist Wasserstoff bevorzugt.

Aliphatische Reste, Alkylreste, Hydroxyalkylreste und Alkoxyreste können geradkettig oder verzweigt sein. Dies gilt auch, wenn sie als Substituenten anderer Reste, z.B. als Substituenten für Arylreste, oder in Verbindung mit anderen Resten auftreten, z.B. als Phenalkyl oder als Alkoxycarbonyl.

Die für $R^2$, $R^3$, $R^4$ und $R^5$ stehenden Alkylreste können gleich oder verschieden sein. In der Regel sind sie alle gleich. Für $R^2$ bis $R^5$ kommen vor allem geradkettige Alkylreste in Betracht. Besonders bevorzugt bedeuten die vier Reste $R^2$, $R^3$, $R^4$ und $R^5$ alle Methyl.

Für $R^6$ ist Alkyl mit 1 bis 4 C-Atomen bevorzugt.

Als für $R^7$ stehende aliphatische Reste kommen insbesondere Alkylreste mit 1 bis 4 C-Atomen in Betracht. Als für $R^7$ stehende aliphatische Reste, die durch Alkoxy mit 1 bis 3 C-Atomen substituiert sind, ist insbesondere der Methoxymethylrest zu nennen. Als für $R^7$ stehende cycloaliphatische Rest kommen vor allem Cycloalkylreste mit 5 bis 7 C-Atomen, insbesondere Cyclopentyl, und bevorzugt Cyclohexyl, in Betracht. Als für $R^7$ stehender bicycloaliphatischer Rest kommt insbesondere das 2,6,6-Trimethylbicyclo-(3.1.1)heptan-3-yl (=Pinanyl-3) in Betracht. Als für $R^7$ stehender tricycloaliphatischer Rest kommt insbesondere das Tricyclo(3.3.1.1$^{3,7}$)decan-1-yl (=Adamantanyl) in Betracht. Als für $R^7$ stehende Alkoxyreste kommen insbesondere Methoxy- und Ethoxy-reste in Betracht. Als für $R^7$ stehender Alkoxycarbonylrest kommt insbesondere der Ethoxycarbonylrest in Betracht. Als für $R^7$ stehende Arylrest sind z.B. $\alpha$- oder $\beta$-Naphthylreste, insbesondere aber der Phenylrest, zu nennen. Als für $R^7$ stehende Aryloxyreste sind z.B. $\alpha$- oder $\beta$-Naphthoxyreste, insbesondere aber der Phenoxyrest, zu nennen. Die für $R^7$ stehenden Arylreste können mono-, di- oder trisubstituiert sein, wobei jedoch auch bei einer Trisubstitution nur maximal 2

Nitrogruppen vorhanden sein können, wie beispielsweise 2-Methyl-4,6-dinitrophenyl und 2-Chlor-6-methyl-4-nitrophenyl. Als Halogensubstituenten für die Arylreste kommen z.B. Chlor und Bromatome in Betracht. Als für $R^7$ stehende substituierte Arylreste sind insbesondere zu nennen: Methylphenyl (= Tolyl), Nitrophenyl und Chlorophenyl, insbesondere 4-Nitrophenyl und 4-Chlorophenyl.

Für $R^7$ sind bevorzugt: Alkylreste mit 1 bis 4 C-Atomen, Alkoxyreste mit 1 oder 2 C-Atomen, Cycloalkylreste mit 5 bis 7 C-Atomen sowie Phenyl. Ganz besonders bevorzugt sind: Methyl, Ethyl, Isopropyl, Ethoxy, Cyclohexyl, Phenyl.

Eine Verbindung der allgemeinen Formel I kann dadurch hergestellt werden, daß eine Verbindung der allgemeinen Formel II

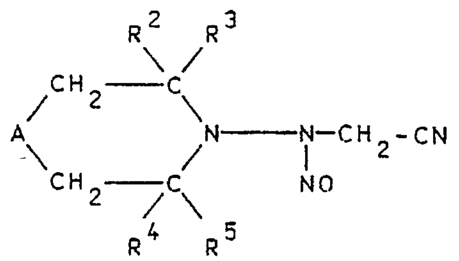

(II)

worin A, $R^2$, $R^3$, $R^4$ und $R^5$ die bereits genannten Bedeutungen besitzen, zu einer Verbindung der allgemeinen Formel Ia

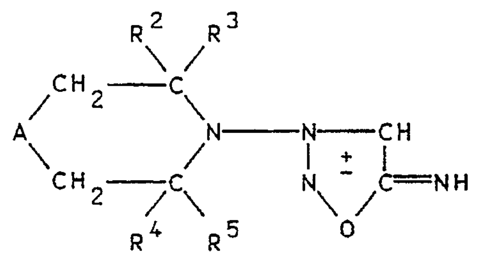

(Ia)

cyclisiert wird und daß diese oder ein Säureadditionssalz davon für den Fall, daß eine Verbindung der Formel I mit $R^1$ = $-COR^7$ hergestellt werden soll, mit einem Acylierungsmittel, das den Rest $-COR^7$ einführt, acyliert wird und die so erhaltene Verbindung gegebenenfalls in ein pharmakologisch annehmbares Säureadditionssalz überführt wird.

Die Cyclisierung der Verbindung II zu den Verbindungen Ia wird in einem geeigneten organischen oder anorganischen Lösungs- oder Dispergiermittel unter Zusatz eines Cyclisierungsmittels, normalerweise bei Temperaturen von 0 bis 40° C, vorzugsweise bei 0 bis 20° C, durchgeführt. Als Cyclisierungsmittel sind solche geeignet, die in wäßriger Lösung einen pH-Wert unter 3 einstellen, also z.B. Mineralsäuren, wie Schwefel-, Salpeter- oder Phosphorsäure, vorzugsweise Chlorwasserstoff, aber auch starke organische Säuren, wie Trifluoressigsäure. Bei der Cyclisierung wird normalerweise das entsprechende Säureadditionssalz der Verbindung Ia erhalten.

Geeignete Lösungs- oder Dispergiermittel sind z.B. Alkohole, insbesondere solche mit 1 bis 6 C-Atomen, vorzugsweise solche mit 1 bis 4 C-Atomen, wie z.B. Methanol, Ethanol, i- und n-Propanol, i-, sec- und tert-Butanol, n-, i-, sec-, tert-Pentanol, n-Hexanol, Cyclopentanol, Cyclohexanol; Ether, insbesondere solche mit 2 bis 8 C-Atomen im Molekül, wie z.B. Diethylether, Methyl-ethyl-ether, Di-n-propyl-ether, Di-isopropyl-ether, Methyl-n-butylether, Ethyl-propyl-ether, Di-butyl-ether, Tetrahydrofuran; 1,4-Dioxan, 1,2-Dimethoxyethan, Bis-β-methoxyethylether; Oligoethylen-glykol-dimethyl-ether, wie z.B. Pentaglyme; Carbonsäurealkylester, insbesondere solche mit 3 bis 8 C-Atomen im Molekül, wie z.B. Essigsäuremethyl- oder -ethylester; Ketone, insbesondere solche mit 3 bis 10 C-Atomen im Molekül, wie z.B. Aceton, Methylethylketon, Methyl-n-propylketon, Diethylketon, 2-Hexanon, 3-Hexanon, Di-n-propylketon, Di-iso-propylketon, Di-isobutylketon, Cyclopentanon, Cyclohexanon, Benzophenon, Acetophenon; aliphatische Kohlenwasserstoffe, wie z.B. Hexan, Heptan, niedrig- und hochsiedende Petrolether; cycloaliphatische Kohlenwasserstoffe, wie z.B. Cyclohexan, Methylcyclohexan, Tetralin, Decalin; aromatische Kohlenwasserstoffe, wie z.B. Benzol, Toluol, o-, m- und p-Xylol, Ethylbenzol; halogenierte aliphatische oder aromatische Kohlenwasserstoff, wie z.B. Methylenchlorid, Chloroform, Tetrachlorkohlenstoff, Chlorbenzol, Dichlorbenzol; Hexamethylphosphorsäuretriamid; Sulfoxide, wie z.B. Dimethyl-sulfoxid; Wasser. Auch Gemische verschiedener Lösungs- oder Dispergiermittel können verwendet werden, beispielsweise Wasser-Methanol oder vorzugsweise Essigsäureethylester-Methanol.

Die Verbindungen der Formel Ia stellen erfindungsgemäße Verbindungen der allgemeinen Formel I für den Fall dar, daß $R^1$ Wasserstoff bedeutet.

Die Acylierung der Verbindung der Formel Ia, die auch in Form eines Säureadditionssalzes vorliegen kann, zur Einführung des Restes $R^1$ = $-COR^7$ kann in an sich bekannter Weise mit einem geeigneten Acylierungsmittel der Formel III durchgeführt werden

$$X-\overset{\overset{O}{\|}}{C}-R^7 \qquad (III)$$

worin X einen nukleophil abspaltbaren Rest darstellt.

In der Formel III bedeutet X z.B. insbesondere Halogen, vorzugsweise -Cl oder -Br; -OH; -O-Alkyl, insbesondere mit 1 bis 5 C-Atomen; -O-Aryl, wobei der Arylrest insbesondere ein Phenylrest ist, der auch mit Alkyl, insbesondere Methyl, und/oder Nitro ein- oder mehrfach substituiert sein kann und beispielsweise ein Tolyl-, Dinitrophenyl- oder Nitrophenyl-Rest ist; $-O-CO-R^7$; -O-CO-O-Alkyl, insbesondere mit 1 bis 5 C-Atomen im Alkylrest, oder den über ein N-Atomen gebundenen Rest eines Azols oder Benzazols mit mindestens 2 N-Atomen im quasi-aromatischen Fünfring.

Die Acylierung wird in einem geeigneten Lösungs- oder Dispergiermittel oder in einem Überschuß des Acylierungsmittels, zweckmäßigerweise unter Rühren, bei Temperaturen von 0 °C bis zur Siedetemperatur des Lösungs-oder Acylierungsmittels, insbesondere 0 bis 50 °C, vorzugsweise von 0 bis 20 °C, durchgeführt.

Das Molverhältnis zwischen der Verbindung der Formel Ia und dem Acylierungsmittel der Formel III beträgt 1 : 1. Zweckmäßigerweise wird das Acylierungsmittel in einem geringen molaren Überschuß eingesetzt. Überschüsse von bis zu 30 mol% sind in der Regel ausreichend, d.h. das Molverhältnis zwischen der Verbindung der Formel Ia und dem Acylierungsmittel der Formel III beträgt normalerweise 1 : (1 bis 1,3), vorzugsweise 1 : (1 bis 1,2).

Falls bei der Acylierungsreaktion eine Säure abgespalten wird, ist der Zusatz eines Säurefängers, wie z.B. eines Alkalihydroxids, wie z.B. Natrium-, Kalium-oder Lithiumhydroxid, eines tertiären organischen Amins, wie z.B. Pyridin oder Triethylamin, eines Alkalicarbonats oder Alkalibicarbonats, wie z.B. Soda oder Natriumbicarbonat, oder eines Alkalisalzes einer schwachen organischen Säure, wie z.B. Natriumacetat, zweckmäßig. Bei der Acylierungsreaktion können auch geeignete Katalysatoren, wie z.B. 4-Dimethylaminopyridin, zugesetzt werden.

Die Verbindungen der Formel III sind Acylierungsmittel und stellen somit z.B. dar: für X = Halogen Säurehalogenide bzw. Halogenameisensäureester, von denen Säurechloride und Chlorameisensäureester bevorzugt sind; für -OH; Carbonsäuren; für -O-Alkyl und -O-Aryl Ester, von denen die Tolyl-, 2,4-Dinitro- oder 4-Nitrophenylester - bevorzugt sind; für $-O-CO-R^7$ Anhydride; für -O-CO-O-Alkyl gemischte Carbonsäure-Kohlensäure-Anhydride; oder heterocyclische Amide oder Azolide, insbesondere von N,N'-Carbonyldiazolen, wie z.B. N,N'-Carbonyldiimidazol, 2,2'-Carbonyl-ditriazol(1,2,3), 1,1'-Carbonyl-ditriazol-(1,2,4), N,N'-Carbonyl-dipyrazol, 2,2'-Carbonyl-ditriazol (vgl. z.B. H.A. Staab, M. Lücking und F.H. Dürr, Chem. Ber. 95, (1962), 1275 ff, H. A. Staab und A. Mannschreck, Chem. Ber. 95, (1962), 1284 ff.; H.A. Staab und W. Rohr, "Synthesen mit heterocyclischen Amiden (Azoliden)" in "Neuere Methoden der Präparativen Organischen Chemie", Band V, Verlag Chemie, 1967, S. 53 ff., insbesondere S. 65 bis 69). Die Acylierungsmittel der Formel III können nach an sich bekannten Verfahren hergestellt werden.

Bei der Verwendung einer Carbonsäure als Acylierungsmittel ist der Zusatz eines Aktivierungsmittels zweckmäßig, das die Aufgabe hat, das Acylierungspotential der Carbonsäure zu erhöhen oder zu aktivieren bzw. die Carbonsäure in situ oder vorzugsweise kurz vor der Umsetzung mit der Verbindung der Formel Ia in ein reaktives Carbonsäurederivat der Formel III zu überführen. Als derartige Aktivierungsmittel sind z.B. geeignet: N,N'-disubstituierte Carbodiimide, insbesondere wenn sie mindestens einen sekundären oder tertiären Alkylrest enthalten, wie z.B. Diisopropyl-, Dicyclohexyl- oder N-Metyhl-N'-tert.butyl-carbodiimid (vgl. Methodicum Chimicum, Verlag G.Thieme, Stuttgart, Bd.6, (1974), S.682/683, und Houben-Weyl, Methoden der Org. Chemie, Bd.8, (1952), S. 521/522); Kohlensäurederivate, wie z.B. Phosgen, Chlorameisensäureester, insbesondere mit 1 bis 5 C-Atomen im Alkylrest (vgl. z.B. Tetrahedron Letters 24 (1983), 3365 bis 3368); Kohlensäureester, wie z.B. N,N'-Disuccinimido-carbonat, Diphthalimido-carbonat, 1,1'-

(Carbonyldioxy)-dibenzo-triazol oder Di-2-pyridyl-carbonat (vgl. z.B. Tetrahedron Letters, Vol.25, No.43, 4943-4946), gegebenenfalls in Anwesenheit geeigneter Katalysatoren, wie z.B. 4-Dimethylaminopyridin. Ferner sind als Aktivierungsmittel N,N′-Carbonyldiazole, wie z.B. N,N′-Carbonyl-diimidazol, 2,2′-Carbonyl-ditriazol(1,2,3), 1,1′-Carbonyl-ditriazol(1,2,4), N,N′-Carbonyl-dipyrazol, 2,2′-Carbonyl-ditetrazol, N,N′-Carbonyl-benzimidazol oder N,N′-Carbonylbenztriazol, geeignet (vgl. z.B. H.A. Staab, M. Lücking und F.H. Dürr, loc. cit; H.A. Staab und A. Mannschreck loc. cit.; H.A. Staab und W. Rohr loc. cit). Als N,N′-Carbonyl-diazol wird häufig das käufliche N,N′-Carbonyl-diimidazol verwendet. Die anderen N,N′-Carbonylazole sind aber aus dem jeweiligen Azol und Phosgen ebenfalls leicht zugänglich.

Als Aktivierungsmittel für die Carbonsäure sind ferner geeignet: Derivate der Oxalsäure, wie z.B. Oxalylchlorid (vgl. z.B. GB-PS 2 139 225) oder N,N′-Oxalyl-diazole wie z.B. 1,1′-Oxalyldi-imidazol, 1,1′-Oxalyldi-1,2,4-triazol und 1,1′-Oxalyldi-1,2,3,4-tetrazol (vgl. z.B. Shizuaka Murata, Bull.Chem. Soc.Jap. 57, 3597-3584)); Methylethylphosphinsäureanhydrid (vgl. z.B. DE-OS 31 01 427); Diphosphortetrajodid (Chem. Lett. 1983, 449); Dialkyldisulfit (Indian J. Chem. 21, 259 (1982)); oder andere reaktive Agentien.

Die Acylierung der Verbindung der Formel Ia mit dem Acylierungsmittel III wird, wie bereits erwähnt, in einem geeigneten Lösungs- oder Dispergiermittel oder in einem Überschuß des Acylierungsmittels durchgeführt. Geeignete Lösungs- oder Dispergiermittel sind z.B. solche, die für die Durchführung der Cyclisierung angegebene worden sind, darüber hinaus auch z.B. Pyridin und Amide, wie z.B. Dimethylformamid. Neben Wasser werden für die Acylierung polare organische Lösungsmittel, wie Dimethylformamid, Dimethylsulfoxid oder Pyridin bevorzugt. Auch Lösungsmittelgemische, wie z.B. ein Gemisch aus Wasser und Methylenchlorid, sind geeignet.

Die substituierten 3-amino-sydnonimine der allgemeinen Formel I bilden mit anorganischen oder organischen Säure Säureadditionssalze. Zur Bildung derartiger Säureadditionssalze sind anorganische oder organische Säure geeignet. Geeignete Säure sind beispielsweise Chlorwasserstoff, Bromwasserstoff, Naphthalindisulfonsäuren, insbesondere Naphthalindisulfonsäure(1,5), Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon-, p-Toluolsulfon-, Zitronen- oder Adipinsäure. Pharmakologisch annehmbare Säureadditionssalze werden bevorzugt. Die Säureadditionssalze können wie üblich durch Vereinigung der Komponenten, zweckmäßigerweise in einem geeigneten Lösungs- oder Verdünnungsmittel, hergestellt werden.

Bei der Synthese der Verbindungen der Formel Ia fallen normalerweise die Säureadditionssalze an. Aus den Säureadditionssalzen können die freien Verbindungen der allgemeinen Formel I bzw. Ia gewünschtenfalls in bekannter Weise, d.h. durch Auflösen oder Suspendieren in Wasser und Alkalischstellen, z.B. mit Natronlauge, und anschließendes Isolieren, gewonnen werden.

Die benötigten Ausgangsverbindungen der allgemeinen Formel II können in an sich bekannter Weise nach der Strecker'schen Aminonitrilsynthese aus Verbindungen der allgemeinen Formel IV

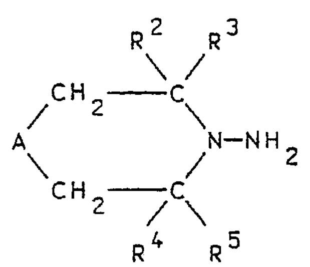

(IV)

worin A, $R^2$, $R^3$, $R^4$ und $R^5$ die bereits genannten Bedeutungen besitzen, durch Umsetzung mit Formaldehyd und Blausäure bzw. Natriumcyanid in einem geeigneten Lösungsmittel, z.B. Wasser, hergestellt werden, wobei zunächst eine Verbindung der allgemeinen Formel V

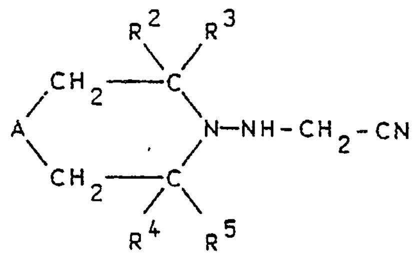

(V)

entsteht, die durch Nitrosierung in die Verbindung II überführt wird. Die Nitrosierung wird in bekannter Weise in einem geeigneten Lösungsmittel, vorzugsweise in Wasser, bei Temperaturen von 0 bis 10 °C durchgeführt. Die salpetrige Säure wird dabei normalerweise aus einem Alkalimetallnitrit, z.B. Natriumnitrit, und Salzsäure erzeugt. Es ist zweckmäßig, die wäßrige Lösung der Verbindung V mit Salzsäure auf einen pH-Wert von 1 bis 3 einzustellen und das Alkalimetallnitrit in Form einer wäßrigen Lösung zu der gerührten und abgekühlten Lösung der Verbindung zuzutropfen.

Die Lösung der dabei erhaltenen Verbindung II kann direkt der Cyclisierungsreaktion unterworfen werden. Normalerweise ist es jedoch angebracht, die Nitrosoverbindung II zunächst in einem geeigneten organischen Lösungsmittel aufzunehmen und in ihm, gegebenenfalls nach Zusatz eines weiteren Lösungsmittels, die Cyclisierung zu der Verbindung der Formel Ia durchzuführen.

Die Verbindungen der allgemeinen Formel IV sind zum Teil bekannt bzw. lassen sich, ausgehend von Verbindungen der allgemeinen Formel VI

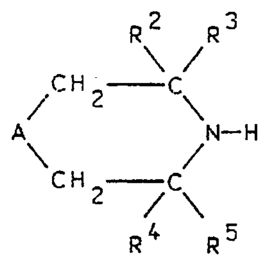

(VI)

dadurch herstellen, daß entweder

a) eine Verbindung der Formel VI zur N-Nitrosoverbindung VII nitrosiert und anschließend, zweckmäßigerweise mit Lithiumaluminiumhydrid, reduziert wird:

(VI)

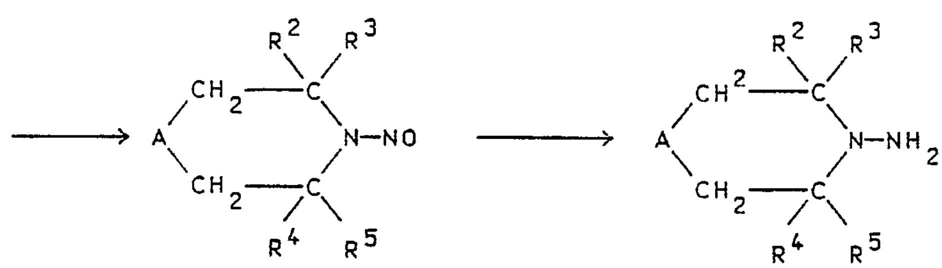

(VII) (IV)

oder

b) in an sich bekannter Weise eine Verbindung der Formel VI mit Kaliumcyanat im sauren Medium in das Harnstoffderivat VIII überführt wird, das dann durch Oxydation mit Natriumhypochlorit nach dem Hoffmann-Abbau in die Verbindung IV überführt wird.

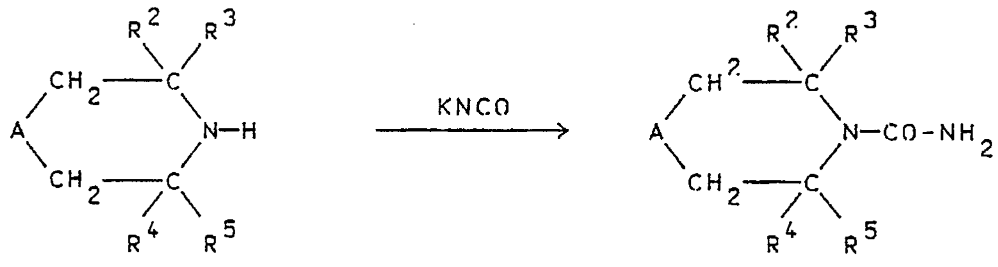


(VI) (VIII)

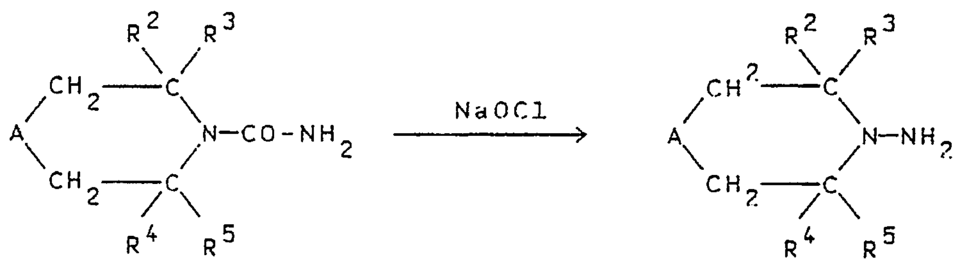


(VIII) (IV)

Die Herstellung der Ausgangsverbindungen der Formel IV und VI ist bekannt. Ausgangsverbindungen der Formel VI lassen sich z.B. aus Verbindungen der allgemeinen Formeln IX oder X

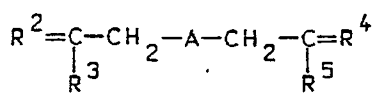

(IX)

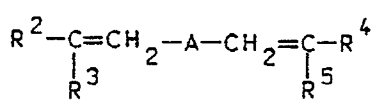

(X)

worin $R^2$ bis $R^5$ und A die bereits angegebenen Bedeutungen besitzen und die nach an sich bekannten Methoden darstellbar sind, durch Ringschluß mit Ammoniak herstellen. Die Umsetzung mit Ammoniak kann bei Temperaturen von 20 bis 150° C, vorzugsweise bei 60 bis 100° C, mit oder ohne Lösungsmittel durchgeführt werden.

Die Herstellung der Ausgangsverbindung IV 1-Amino-2,2,6,6-tetramethyl-piperidin ist z.B. beschrieben von J.R.Roberts and K.U.Ingold in J.A.C.S. 95, (1973), 3229 sowie von William D. Hinsberg III, Peter G. Schultz und Peter B. Dervan in J.A.C.S. 104, (1982), 771, die auch die Herstellung der Ausgangsverbindung 1-Amino-2,2,5,5-tetramethylpyrrolidin auf Seite 772 beschreiben. Die Herstellung der Ausgangsverbindung IV 1-Amino-1-aza-2,2,6,6-tetramethyl-4-thiacyclohexan-4,4-dioxid ist beschrieben in Beispiel 87 der DE-A-2351865. In der DE-A-23 51 865 sind auch 2,2,6,6-tetraalkylsubstituierte 1-Aza-4-thiacyclohexan-4,4-dioxide beschrieben. Die Herstellung von 3,3,5,5-tetraalkylsubstituierten Morpholinen als Ausgangsverbindungen IV ist beschrieben von J.T. Lai in Synthesis (1984), 122-123. Andere Ausgangsverbindungen der Formeln IV und VI können analog der vorerwähnten Angaben hergestellt werden.

Die Verbindung der allgemeinen Formel I und ihre pharmakologisch annehmbaren Säureadditionssalze besitzen wertvolle pharmakologische Eigenschaften. Besonders ausgeprägt ist ihre Wirkung auf das Herz-Kreislauf-System. Verglichen mit bekannten, in 3-Stellung substituierten Sydnoniminverbindungen, z.B. solchen der EP-B-59356, sowie der im Handel befindlichen strukturähnlichen Verbindung Molsidomin, besitzen sie überraschenderweise eine wesentlich längere Wirkungsdauer. Sie senken beispielsweise den Blutdruck ebenso wie den Pulmonalarteriendruck und den linksvertrikulären enddiastolischen Druck und tragen so zu einer Entlastung der Herztätigkeit im Sinne einer antianginösen Wirkung bei, ohne dabei eine reflektorische Tachykardie zu provozieren.

Die Verbindungen der Formel I und ihre pharmakologisch annehmbaren Säureadditionsssalze können

daher am Menschen als Heilmittel fur sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der Formel I oder eines Säureadditionssalzes davon, neben üblichen pharmazeutisch einwandfreien Träger- und Zusatzstoffen enthalten.

Die Heilmittel können oral, z.B. in Form von Pillen, Tabletten, Lacktabletten, Dragees, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Zur Herstellung der pharmazeutischen Präparate können pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze verwenden. Trägerstoffe für Weichgelatinekapseln und Suppositorien sind z.B. Fette, Wachse, halbfeste und flüssige Polyole, natürliche oder gehärtete Öle. Als Trägerstoffe für die Herstellung von Lösungen und Sirupen eignen sich z.B. Wasser, Saccharose, Invertzucker, Glukose, Polyole. Als Trägerstoffe für die Herstellung von Injektionslösungen eignen sich z.B. Wasser, Alkohole, Glyzerin, Polyole oder pflanzliche Öle.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmitel oder Antioxidantien enthalten. Sie können auch zwei oder mehrere Verbindungen der Formel I oder ihrer pharmakologisch annehmbaren Säureadditionssalze und noch andere therapeutisch wirksame Stoffe enthalten.

Derartige andere therapeutisch wirksame Substanzen sind beispielsweise: $\beta$-Rezeptorenblocker, wie z.B. Propranolol, Pindolol, Metoprolol; Vasodilatatoren, wie z.B. Carbochromen; Beruhigungsmittel, wie z.B. Barbitursäurederivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Prazosin, Clonidin, Rauwolfia-Alkaloide; Mittel, die den Fettsäurespiegel im Blut senken, wie z.B. Benzafibrat, Fenofibrat; Mittel für die Thromboseprophylaxe, wie z.B. Phenprocoumon.

Die Verbindungen der Formel I, ihre pharmakologisch annehmbaren Säureadditionsssalze und pharmazeutische Präparate, welche die Verbindungen der Formel I oder ihre pharmakologisch annehmbaren Säureadditionssalze als Wirkstoffe enthalten, können am Menschen bei der Bekämpfung bzw. Vorbeugung von Erkrankungen des kardiovaskulären Systems verwendet werden, beispielsweise als antihypertensive Heilmittel bei den verschiedenen Formen des Bluthochdrucks, bei der Bekämpfung bzw. Vorbeugung von Angina pectoris usw. Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis von etwa 0,5 bis 100 mg, vorzugsweise 1 bis 20 mg, angemessen. Auch bei anderen Applikationsformen liegt die Tagesdosis, wegen der guten Resorption der Wirkstoffe, in ähnlichen Mengenbereichen, d.h. im allgemeinen ebenfalls bei 0,5 bis 100 mg/Mensch. Die Tagesdosis wird normalerweise in mehrere, z.B. 2 bis 4 Teilverabreichungen aufgeteilt.

Die pharmakologische Wirkung der verbindungen der Formel I wurde nach einer modifizierten Methode von Godfraind and Kaba (Arch.Int. Pharmacodyn. Ther. 196, (Suppl) 35 bis 49, 1972) und von Schüman et al (Naunyn-Schmiedeberg's Arch. Pharmacol. 289, 409 bis 418, 1975) ermittelt. Dabei werden Spiralstreifen der Arteria pulmonalis des Meerschweinchens nach Äquilibrierung in calciumfreier Tyrodelösung mit 40 mmol/l Kalium depolarisiert. Ein Zusatz von 0,5 mmol/l $CaCl_2$ löst dann eine Kontraktion aus. Die relaxierende Wirkung der Prüfsubstanz wird durch kumulative Zugabe in 1/2 log 10 abgestuften Konzentrationen ermittelt. Aus der Konzentationswirkungskurve (Abszisse: -log mol/l Prüfsubstanz, Ordinate: % Hemmung der maximalen Kontraktion, Mittelwert von 4 bis 6 Gefäßstreifen) wird die Konzentration der Prüfsubstanz ermittelt, welche die Kontraktion um 50 % hemmt (= $IC_{50}$, mol/l). In der folgenden Tabelle sind die so erhaltenen $IC_{50}$-Werte angegeben. Wie der Vergleich mit dem $IC_{50}$-Wert $3 \cdot 10^{-4}$ für die bekannte Verbindung Molsidomin (N-Ethoxycarbonyl-3-morpholino-sydnonimin), vgl. DE-B-16 95 897, ergibt, liegen die Werte für die Verbindungen der Formel I erheblich günstiger.

| IC50-Werte in mol/l | |
|---|---|
| | $IC_{50}$ |
| a) 3-(2,2,6,6-Tetramethylpiperidino)-sydnonimin-hydrochlorid | $3.10^{-5}$ |
| b) 3-(2,2,6,6-Tetramethyl-4-isopropylpiperazin-1-yl)-sydnonimin-dihydrochlorid | $3.10^{-6}$ |
| c) 3-(3,3,5,5-Tetramethyl-morpholino)-sydnonimin-hydrochlorid | $1.10^{-4}$ |
| d) N-Ethoxycarbonyl-3-morpholino-sydnonimin | $3.10^{-4}$ |
| a bis c: Erfindungsgemäße Verbindungen<br>d: Vergleichsverbindung Molsidomin | |

Beispiel 1
3-(2,2,6,6-Tetramethylpiperidino)-sydnonimin-hydrochlorid

a) N-(2,2,6,6-Tetramethylpiperidino)-aminoacetonitril

Zur eisgekühlten Mischung von 17,5 g 1-Amino-2,2,6,6-tetramethylpiperidin, 11 g konzentrierter Salzsäure und 50 ml Wasser wird die Lösung von 8,7 g Kaliumcyanid in 20 ml Wasser gegeben und der pH-Wert mit Salzsäure auf 6,5 eingestellt. Dann werden 9,65 g einer 39%igen wäßrigen Formaldehydlösung zugesetzt und die Reaktionsmischung wird 3 h bei 0°C und weitere 3 h bei Raumtemperatur gerührt. Das Produkt wird mit Essigsäureethylester extrahiert, die Essigsäureethylesterphase mit verdünntem Eisessig gewaschen und über Natriumsulfat getrocknet. Nach dem Einengen verbleibt ein teilweise kristalliner Rückstand von 10,7 g N-(2,2,6,6-Tetramethylpiperidino)-aminoacetonitril, der ohne weitere Reinigung weiterverarbeitet wird.

b) 3-(2,2,6,6-Tetramethylpiperidino)-sydnonimin-hydrochlorid

Zur Mischung von 10,7 g der vorstehend unter a) beschriebenen Verbindung, 50 ml Wasser, 50 ml Essigsäureethylester und 6 g konzentrierte Salzsäure wird die Lösung von 4,2 g Natriumnitrit in 10 ml Wasser getropft und nach 5 h Rühren bei Raumtemperatur die organische Phase abgetrennt, mit 15 ml Methanol verdünnt und im Eisbad gerührt. Nach dem Einleiten von überschüssigem Chlorwasserstoff wird 15 h bei Raumtemperatur nachgerührt und im Wasserstrahl-Vakuum eingeengt. Der Rückstand kristallisiert beim Verrühren mit Diethylester. Er wird abgesaugt und mit Essigsäureethylester gewaschen.

Ausbeute: 8,6 g 3-(2,2,6,6-Tetramethylpiperidino)-sydnonimin-hydrochlorid mit Schmelzpunkt: 170°C (Zersetzung).

Beispiel 2
3-(2,2,6,6-Tetramethylpiperidino)-N-ethoxycarbonyl-sydnonimin

Zur eisgekühlten Lösung von 5 g 3-(2,2,6,6-Tetramethylpiperidino)-sydnonimin-hydrochlorid gemäß dem Beispiel 1 b) in 20 ml Wasser werden 3,5 g Natriumbicarbonat gegeben und anschließend die Lösung von 2,5 g Chlorameisensäurethylester in 20 ml Methylenchlorid zugefügt und die Mischung 4 h bei Raumtemperatur gerührt. Die organische Phase wird abgetrennt, getrocknet und eingeengt. Der Rückstand wird mit Petrolether verrieben und abgesaugt.

Ausbeute: 4,2 g 3-(2,2,6,6-Tetramethylpiperidino)-N-ethoxycarbonyl-sydnonimin mit Schmelzpunkt 74 bis 75°C.

Beispiel 3
3-(3,3,5,5-Tetramethyl-morpholino)-sydnonimin-hydrochlorid

a) 4-Nitroso-3,3,5,5-tetramethyl-morpholin

Die Lösung von 27 g 3,3,5,5-Tetramethyl-morpholin-hydrochlorid (hergestellt nach Synthsis (1984), 122) in 80 ml Wasser wird mit 15,6 g Natriumnitrit versetzt und 3 h auf 80°C erwärmt. Nach dem Erkalten wird die Nitrosoverbindung mit Diethylether extrahiert. Nach dem Trocknen und Einengen der Etherphase kristallisieren 22,2 g der Verbindung aus. Schmelzpunkt: 47°C.

b) 4-Amino-3,3,5,5-tetramethyl-morpholin

Zur erwärmten Lösung von 17,2 g 4-Nitroso-3,3,5,5-tetramethyl-morpholin in 50 ml Tetrahydrofuran und 100 ml Dibutylether wird etwa 20 % der Lösung von 5 g Lithiumalanat in 50 ml Tetrahydrofuran zugetropft. Das Tetrahydrofuran wird abdestilliert und die Innentemperatur auf 99 °C erhöht. Dann wird die restliche Lithiumalanat-Lösung zugetropft und die Mischung noch 1 h auf 70 °C gehalten. Nach dem Abkühlen der Mischung im Eisbad wird vorsichtig mit 10 ml Wasser und mit 10 ml 15%iger Natronlauge versetzt. Nach dem Absaugen wird das Filtrat erst mit Wasser und dann mit 100 ml 1 N Salzsäure ausgeschüttelt. Die wäßrige HCl-Phase wird dann noch einmal mit Diethylether ausgeschüttelt und in der nächsten Stufe eingesetzt.

c) N-(3,3,5,5-Tetramethyl-morpholino)-aminoacetonitril

Zu der HCl-Phase aus der vorhergehenden Stufe wird die Lösung von 8,7 g Kaliumcyanid in 20 ml Wasser gegeben und der pH-Wert mit Salzsäure auf 6,5 eingestellt. Dann werden 9,5 g einer 39%igen wäßrigen Formaldehydlösung zugesetzt und die Reaktionsmischung 3 h bei 0 °C und weitere 3 h bei Raumtemperatur gerührt. Das Produkt wird mit Essigsäureethylester extrahiert, die Essigsäureethylesterphase mit verdünntem Eisessig gewaschen und über Natriumsulfat getrocknet. Nach dem Einengen verbleibt ein teilweise kristalliner Rückstand von N-(3,3,5,5-Tetramethylmorpholino)-aminoacetonitril, der ohne weitere Reinigung weiterverarbeitet wird.

d) 3-(3,3,5,5-Tetramethyl-morpholino)-sydnonimin-hydrochlorid

Zur Mischung der vorstehend unter c) beschriebenen Verbindung, 50 ml Wasser, 50 ml Essigsäureethylester und 6 g konzentrierte Salzsäure wird die Lösung von 4,2 g Natriumnitrit in 10 ml Wasser getropft und nach 5 h Rühren bei Raumtemperatur die organische Phase abgetrennt, mit 15 ml Methanol verdünnt und im Eisbad gerührt. Nach dem Einleiten von überschüssigem Chlorwasserstoff wird 15 h bei Raumtemperatur nachgerührt und im Wasserstrahl-Vakuum eingeengt. Der Rückstand kristallisiert beim Verrühren mit Diethylester. Er wird abgesaugt und mit Essigsäureethylester gewaschen.

Ausbeute: 3,9 g 3-(3,3,5,5-Tetramethylmorpholino)-sydnonimin-hydrochlorid vom Schmelzpunkt: 132-133 °C (Zersetzung).

Beispiel 4

3-(2-2,6,6-Tetramethyl-4-isopropyl-piperazin-1-yl)-sydnonimin-dihydrochlorid

a) 1-Nitroso-2,2,6,6-Tetramethyl-4-isopropyl-piperazin

15,6 g 1-Isopropyl-3,3,5,5-tetramethyl-piperazin werden analog der Vorschrift des Beispiels 3a umgesetzt. Bei der Aufarbeitung werden 14,8 g eines Öls erhalten.

b) 1-Amino-2,2,6,6-tetramethyl-4-isopropyl-piperazin

Das in der vorstehenden Stufe erhaltene 1-Nitroso-2,2,6,6-Tetramethyl-4-isopropyl-piperazin wird analog Beispiel 3b umgesetzt. Die dabei erhaltene wäßrige HCl-Phase wird in der nächsten Stufe eingesetzt.

c) N-(2,2,6,6-Tetramethyl-4-isopropyl-piperazin-1-yl)-amino-acetonitril

Die wäßrige HCl-Phase aus der vorhergehenden Stufe wird analog Beispiel 3c weiterverarbeitet und das dabei erhaltene Produkt in der folgenden Stufe eingesetzt.

d) 3-(2,2,6,6-Tetramethyl-4-isopropyl-piperazin-1-yl)-sydnonimin-dihydrochlorid

Das in der vorhergehenden Stufe erhaltene Produkt wird analog Beispiel 3d) weiterverarbeitet.

Ausbeute: 4,6 g 3-(2,2,6,6-Tetramethyl-4-isopropyl-piperazino)-sydnonimin-dihydrochlorid vom Schmelzpunkt 142 °C (Zersetzung).

e) 1-Isopropyl-3,3,5,5-tetramethyl-piperazin

Das in der Stufe a) als Ausgangsprodukt benötigte 1-Isopropyl-3,3,5,5-tetramethyl-piperazin kann wie folgt hergestellt werden:
3,9 g (0,103 mol) Lithiumaluminiumhydrid werden unter Rühren und Kühlen unter Stickstoffatmosphäre in 100 ml wasserfreies Tetrahydrofuran eingetragen. Hierzu wird eine Lösung von 19,8 g (0,1 mol) 1-Isopropyl-3,3,5,5-tetramethyl-2-piperazinon (erhältlich z.B. nach Synthesis 1981, S. 40, oder J. Organ. Chem., 45 - (1980), 754-59) in 75 ml wasserfreiem Tetrahydrofuran bei Raumtemperatur getropft, wobei die Reaktionstemperatur auf 60 °C ansteigt. Dann wird über Nacht bei Raumtemperatur gerührt. Nach dem Zutropfen von ca. 10 ml Wasser wird 2 Stunden nachgerührt, dann vom Niederschlag abgesaugt. Das Filtrat wird über Pottasche getrocknet, im Vakuum eingeengt und das als farbloses Öl zurückbleibende Rohprodukt ohne zusätzliche Reinigung weiter verarbeitet.

Ausbeute: 16 g $n_D^{25}$ 1,4497

Analog können auch andere benötigte Ausgangsprodukte hergestellt werden.

Beispiel 5:
3-(3,3,5,5-Tetramethyl-1,4-thiazin-1,1-dioxid-4-yl)-sydnonimin-hydrochlorid

Die Verbindung wird ausgehend von 18 g 4-Amino-3,3,5,5-Tetramethyl-1,4-thiazin-1,1-dioxid (hergestellt nach Beispiel 8 der DE-A-23 51 865) analog Beispiel 3 hergestellt.

Ausbeute: 6,2 g 3-(3,3,5,5-Tetramethyl-1,4-thiazin-1,1-dioxid-4-yl)-sydnonimin-hydrochlorid vom Schmelzpunkt 177 °C (Zersetzung).

Beispiel 6:
3-(2,2,6,6-Tetramethyl-4-tert.butyl-piperazin-1-yl)-sydnonimin-dihydrochlorid

Die Verbindung wird analog Beispiel 4 hergestellt.
Schmelzpunkt: 110-112 °C (Zersetzung)

Beispiel 7:
3-(2,2,6,6-Tetramethyl-4-(2-hydroxy-1,1-dimethyl-ethyl)-piperazin-1-yl)-sydnonimin-dihydrochlorid

Die Verbindung wird analog Beispiel 4 hergestellt.
Schmelzpunkt: 124-126 °C (Zersetzung).

Beispiel 8:
3-(2,2,6,6-Tetramethyl-4-benzyl-piperazin-1-yl)-sydnonimin-dihydrochlorid

Die Verbindung wird analog Beispiel 4 hergestellt.
Schmelzpunkt: 156-158 °C (Zersetzung)

Beispiel 9:
3-(2,2,6,6-Tetramethyl-4-isopropyl-piperazin-1-yl)-N-ethoxycarbonyl-sydnonimin

Die Verbindung wird analog Beispiel 2 hergestellt.
Schmelzpunkt: 98-99 °C (Zersetzung)

3,3,5,5-Tetraalkylsubstituierte Piperazine lassen sich z.B. nach dem in Beispiel 4e angegebenen Verfahren herstellen.

Beispiel 10:
3-(2,2,6,6-Tetramethyl-4-isopropyl-piperazin-1-yl)-N-acetyl-sydnonimin

Die Verbindung wird analog Beispiel 2 hergestellt.
Schmelzpunkt: 121-123 °C (Zersetzung)

Beispiel 11:
3-(2,2,6,6-Tetramethyl-4-isopropyl-piperazin-1-yl)-N-benzoyl-sydnonimin

Die Verbindung wird analog Beispiel 2 hergestellt.
Schmelzpunkt: 133-135 °C (Zersetzung)

Beispiel 12:
3-(2,2,6,6-Tetramethyl-piperidino)-N-cyclohexylcarbonyl-sydnonimin

Die Verbindung wird analog Beispiel 2 hergestellt.
Schmelzpunkt: 107-108 °C (Zersetzung)

Beispiel 13:
3-(3,3,5,5-Tetramethyl-morpholino)-N-isobutyryl-sydnonimin

Die Verbindung wird analog Beispiel 2 hergestellt.
Schmelzpunkt: 88-89 °C (Zersetzung)
In den nachfolgenden Beispielen A bis E werden pharmazeutische Präparate beschrieben.

Beispiel A

Gelatineweichkapseln, enthaltend 5 mg Wirkstoff pro Kapsel:

| | pro Kapsel |
|---|---|
| Wirkstoff | 5 mg |
| Aus Kokosfett fraktioniertes Triglycerid-Gemisch | 150 mg |
| Kapselinhalt | 155 mg |

Beispiel B

Injektionslösung, enthaltend 1 mg Wirkstoff pro ml:

| | pro ml |
|---|---|
| Wirkstoff | 1,0 mg |
| Polyethylenglycol 400 | 0,3 ml |
| Natriumchlorid | 2,7 mg |
| Wasser zu Injektionszwecken | ad 1 ml |

Beispiel C

Emulsion, enthaltend 3 mg Wirkstoff pro 5 ml

| | pro 100 ml Emulsion |
|---|---|
| Wirkstoff | 0,06 g |
| Neutralöl | q.s. |
| Natriumcarboxymethylcellulose | 0,6 g |
| Polyoxyethylen-stearat | q.s. |
| Glycerin rein | 0,2 bis 2,0 g |
| Geschmacksstoff | q.s. |
| Wasser (entsalzt oder destilliert) | ad 100 ml |

Beispiel D

Rektale Arzneiform, enthaltend 4 mg Wirkstoff pro Suppositorium

| | pro Suppositorium |
|---|---|
| Wirkstoff<br>Suppositoriengrundmasse | 4 mg<br>ad 2 g |

Beispiel E

Tabletten, enthaltend 2 mg Wirkstoff pro Tablette

| | pro Tablette |
|---|---|
| Wirkstoff | 2 mg |
| Lactat (feingemahlen) | 2 mg |
| Maisstärke (weiß) | 150 mg |
| Milchzucker | 60 mg |
| Mikrokristalline Cellulose | 50 mg |
| Polyvinylpyrrolidon | 20 mg |
| Magnesiumstearat | 2 mg |
| Natriumcarboxymethylstärke | 25 mg |
| | 311 mg |

**Patentansprüche**
**Patentansprüche für folgende Vertragsstaaten : DE, FR, GB, CH, IT, BE, NL, AT, SE**

1. Substituierte 3-Aminosydnonimine der allgemeinen Formel I

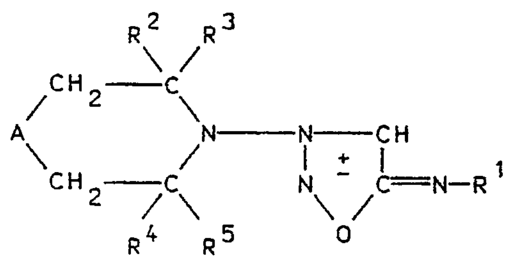

(I)

und ihre pharmakologisch annehmbaren Säureadditionssalze, worin
A den Rest $-CH_2-$, -O-, $-S(O)_n-$, $-N(R^6)-$ oder eine direkte Bindung;
$R^1$ Wasserstoff oder den Rest $-COR^7$;
$R^2$, $R^3$, $R^4$, $R^5$ Alkyl mit 1 bis 4 C-Atomen;
n die Zahl 0, 1 oder 2;
$R^6$ Alkyl mit 1 bis 4 C-Atomen; Hydroxyalkyl mit 1 bis 4 C-Atomen; Phenylalkyl mit 1 bis 4 C-Atomen im Alkylrest;
$R^7$ einen aliphatischen Rest mit 1 bis 4 C-Atomen, der auch durch Alkoxy mit 1 bis 3 C-Atomen substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen durch 1 bis 3 Halogenatome und/oder 1 bis 3 Alkylreste mit 1 bis 3 C-Atomen und/oder 1 bis 3 Alkoxyreste mit 1 bis 3 C-Atomen und/oder 1 oder 2 Nitrogruppen mono-, di- oder trisubstituierten Arylrest mit 6 bis 10 C-Atomen;
bedeuten.

2. Substituierte 3-Aminosydnonimine nach Anspruch 1, dadurch gekennzeichnet, daß $R^1$ Wasserstoff bedeutet.

3. Substituierte 3-Aminosydnonimine nach Anspruch 1 und/oder 2, dadurch gekennzeichnet, daß $R^2$, $R^3$, $R^4$ und $R^5$ Methyl bedeuten.

4. Substituierte 3-Aminosydnonimine nach einem oder mehreren der Ansprüche 1 bis 3, dadurch gekennzeichnet, daß A $-CH_2-$, -O- oder $-N(R^6)-$ bedeutet.

5. 3-(2,2,6,6-Tetramethylpiperidino)-sydnonimin und seine pharmakologisch annehmbaren Säureadditionssalze.

6. 3-(2,2,6,6-Tetramethyl-4-isopropyl-piperazin-1-yl)-sydnonimin und seine pharmakologisch annehmbaren Säureadditionssalze, insbesondere sein Dihydrochlorid.

7. Verfahren zur Herstellung der in einem oder mehreren der Ansprüche 1 bis 4 angegebenen Verbindungen der Formel I oder der in den Ansprüchen 5 und 6 angegebenen Verbindungen, dadurch gekennzeichnet, daß eine Verbindung der allgemeinen Formel II

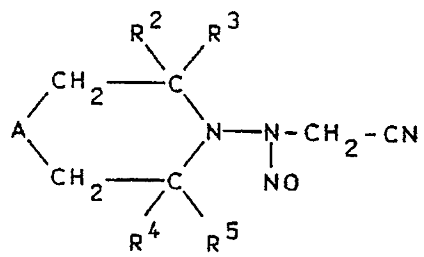

(II)

worin A, $R^2$, $R^3$, $R^4$ und $R^5$ die in einem oder mehreren der Ansprüche 1 bis 5 angegebenen Bedeutungen besitzen, zu einer Verbindung der Formel Ia

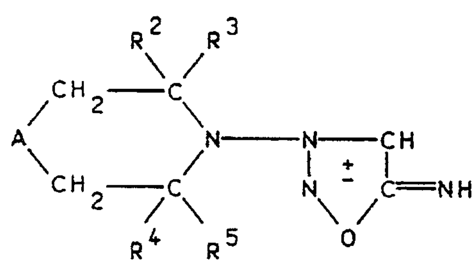

(Ia)

die auch in Form eines Säureadditionssalzes vorliegen kann, cyclisiert wird und gegebenenfalls aus dem Säureadditionssalz die freie Verbindung isoliert wird und daß für den Fall, daß eine Verbindung der Formel I mit $R^1$ = $-COR^7$ hergestellt wird, die Verbindung der Formel Ia oder ein Säureadditionssalz davon mit einem Acylierungsmittel acyliert wird, das den Rest $-COR^7$ einführt und die erhaltene Verbindung gegebenenfalls ein ein Säureadditionssalz überführt wird.

8. Verfahren nach Anspruch 7, dadurch gekennzeichnet, daß die Cyclisierung in einem Lösungs- oder Dispergiermittel bei Temperaturen von 0 bis 40 °C, vorzugsweise 0 bis 20 °C, mit Hilfe von Cyclisierungsmitteln, die in wäßriger Lösung einen pH-Wert unter 3 einstellen, durchgeführt wird.

9. Verwendung eines oder mehrerer substituierter 3-Amino-sydnonimine und/oder eines oder mehrerer ihrer pharmakologisch annehmbaren Säureadditionssalze der Ansprüche 1 bis 6 als pharmakologische Wirkstoff zusammen mit einem oder mehreren pharmakologisch annehmbaren Träger-und/oder Zusatzstoffen und gegebenenfalls noch einem oder mehreren anderen therapeutisch wirksamen Stoffen zur Herstellung pharmazeutischer Präparate, die zur Anwendung bei der Bekämpfung und Vorbeugung cardiovaskulärer Erkrankungen bestimmt sind.

10. Pharmazeutisches Präparat, dadurch gekennzeichnet, daß es eine oder mehrere Verbindungen der Ansprüche 1 bis 6 oder ein oder mehrere Säureadditionssalze davon als Wirkstoffe zusammen mit

pharmakologisch annehmbaren Träger- und/oder Zusatzstoffen und gegebenenfalls noch einen oder mehrere andere therapeutisch wirksame Stoffe enthält.

**Patentansprüche für folgenden Vertragsstaat : ES**

1. Verfahren zur Herstellung substituierter 3-Aminosydnonimine der allgemeinen Formel I

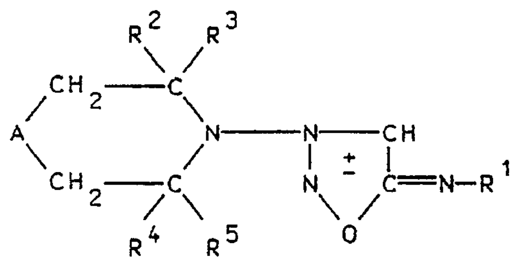

(I)

und ihrer pharmakologisch annehmbaren Säureadditionssalze, worin
A den Rest $-CH_2-$, -O-, $-S(O)_n-$, $-N(R^6)-$ oder eine direkte Bindung;
$R^1$ Wasserstoff oder den Rest $-COR^7$;
$R^2$, $R^3$, $R^4$, $R^5$ Alkyl mit 1 bis 4 C-Atomen;
n die Zahl 0, 1 oder 2;
$R^6$ Alkyl mit 1 bis 4 C-Atomen, Hydroxyalkyl mit 1 bis 4 C-Atomen; Phenylalkyl mit 2 bis 4 C-Atomen im Alkylrest;
$R^7$ einen aliphatischen Rest mit 1 bis 4 C-Atomen, der auch durch Alkoxy mit 1 bis 3 C-Atomen substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen durch 1 bis 3 Halogenatome und/oder 1 bis 3 Alkylreste mit 1 bis 3 C-Atomen und/oder 1 bis 3 Alkoxyreste mit 1 bis 3 C-Atomen und/oder 1 oder 2 Nitrogruppen mono-, di- oder trisubstituierten Arylrest mit 6 bis 10 C-Atomen;
bedeuten,
dadurch gekennzeichnet, daß eine Verbindung der allgemeinen Formel II

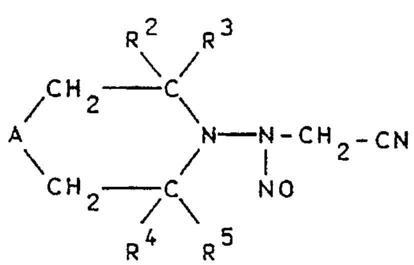

(II)

worin A, $R^2$, $R^3$, $R^4$ und $R^5$ die in einem oder mehreren der Ansprüche 1 bis 5 angegebenen Bedeutungen besitzen, zu einer Verbindung der Formel Ia

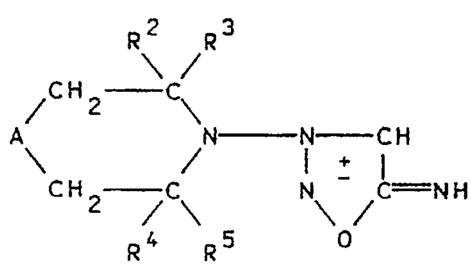

(Ia)

die auch in Form eines Säureadditionssalzes vorliegen kann, cyclisiert wird und gegebenenfalls aus

dem Säureadditionssalz die freie Verbindung isoliert wird und daß für den Fall, daß eine Verbindung der Formel I mit $R^1$ = $-COR^7$ hergestellt wird, die Verbindung der Formel Ia oder ein Säureadditionssalz davon mit einem Acylierungsmittel acyliert wird, das den Rest $-COR^7$ einführt und die erhaltene Verbindung gegebenenfalls in ein Säureadditionssalz überführt wird.

2. Verfahren nach Anspruch 1, dadurch gekennzeichnet, daß die Verbindung Ia ohne Acylierung isoliert oder ohne Acylierung in ein Säureadditionssalz überführt wird.

3. Verfahren nach Anspruch 1 und/oder 2, dadurch gekennzeichnet, daß eine Verbindung der Formel II eingesetzt wird, in der $R^2$, $R^3$, $R^4$ und $R^5$ Methyl bedeuten.

4. Verfahren nach einem oder mehreren der Ansprüche 1 bis 3, dadurch gekennzeichnet, daß eine Verbindung der Formel II eingesetzt wird, in der A $-CH_2-$ bedeutet.

5. Verfahren nach einem oder mehreren der Ansprüche 1 bis 3, dadurch gekennzeichnet, daß eine Verbindung der Formel II eingesetzt wird, in der A -O- bedeutet.

6. Verfahren nach einem oder mehreren der Ansprüche 1 bis 3, dadurch gekennzeichnet, daß eine Verbindung der Formel II eingesetzt wird, in der A $-N(R^6)-$ bedeutet.

7. Verfahren nach Anspruch 1, dadurch gekennzeichnet, daß eine Verbindung der Formel II eingesetzt wird, in der $R^2$, $R^3$, $R^4$ und $R^5$ Methyl und A $-N(-CH(CH_3)_2)-$ bedeuten, und daß die Verbindung Ia ohne Acylierung isoliert oder ohne Acylierung in ein Säureadditionssalz, insbesonderes das Dihydrochlorid, überführt wird.

8. Verfahren nach Anspruch 1, dadurch gekennzeichnet, daß eine Verbindung der Formel II eingesetzt wird, in der $R^2$, $R^3$, $R^4$ und $R^5$ Methyl und A - $CH_2$- bedeuten, und daß die Verbindung Ia ohne Acylierung isoliert oder ohne Acylierung in ein Säureadditionssalz überführt wird.

9. Verwendung eines oder mehrerer substituierter 3-Aminosydnonimine der allgemeinen Formel I

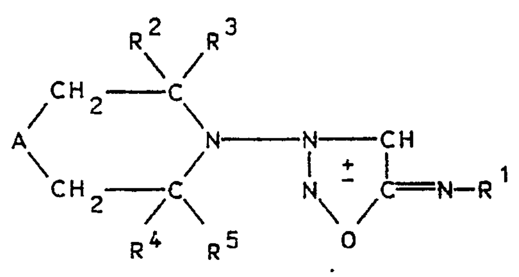

(I)

und/oder eines oder mehrerer ihrer pharmakologisch annehmbaren Säureadditionssalze, worin
A den Rest $-CH_2-$, -O-, $-S(O)_n-$, $-N(R^6)-$ oder eine direkte Bindung;
$R^1$ Wasserstoff oder den Rest $-COR^7$;
$R^2$, $R^3$, $R^4$, $R^5$ Alkyl mit 1 bis 4 C-Atomen;
n die Zahl 0, 1 oder 2;
$R^6$ Alkyl mit 1 bis 4 C-Atomen, Hydroxyalkyl mit 1 bis 4 C-Atomen; Phenylalkyl mit 2 bis 4 C-Atomen im Alkylrest;
$R^7$ einen aliphatischen Rest mit 1 bis 4 C-Atomen, der auch durch Alkoxy mit 1 bis 3 C-Atomen substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen durch 1 bis 3 Halogenatome und/oder 1 bis 3 Alkylreste mit 1 bis 3 C-Atomen und/oder 1 bis 3 Alkoxyreste mit 1 bis 3 C-Atomen und/oder 1 oder 2 Nitrogruppen mono-, di- oder trisubstituierten Arylrest mit 6 bis 10 C-Atomen;
bedeuten, zusammen mit pharmakologisch annehmbaren Träger- und/oder Zusatzstoffen und gegebenenfalls noch einem oder mehreren anderen therapeutisch wirksamen Stoffen zur Herstellung pharmazeutischer Präparate, die zur Anwendung bei der Bekämpfung und Vorbeugung cardiovaskulärer

Erkrankungen bestimmt sind.

**10.** Verfahren zur Herstellung eines pharmazeutischen Präparats, dadurch gekennzeichnet, daß ein oder mehrere substituierte 3-Aminosydnonimine der allgemeinen Formel I

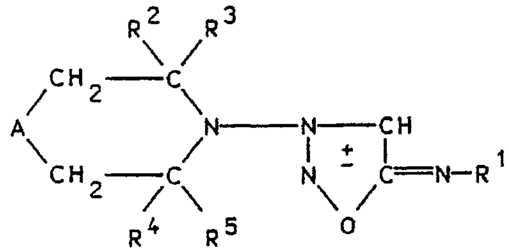

(I)

und/oder ein oder mehrere ihrer pharmakologisch annehmbaren Säureadditionssalze, worin
A den Rest $-CH_2-$, -O-, $-S(O)_n-$, $-N(R^6)-$ oder eine direkte Bindung;
$R^1$ Wasserstoff oder den Rest $-COR^7$;
$R^2$, $R^3$, $R^4$, $R^5$ Alkyl mit 1 bis 4 C-Atomen;
n die Zahl 0, 1 oder 2;
$R^6$ Alkyl mit 1 bis 4 C-Atomen, Hydroxyalkyl mit 1 bis 4 C-Atomen; Phenylalkyl mit 2 bis 4 C-Atomen im Alkylrest;
$R^7$ einen aliphatischen Rest mit 1 bis 4 C-Atomen, der auch durch Alkoxy mit 1 bis 3 C-Atomen substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen durch 1 bis 3 Halogenatome und/oder 1 bis 3 Alkylreste mit 1 bis 3 C-Atomen und/oder 1 bis 3 Alkoxyreste mit 1 bis 3 C-Atomen und/oder 1 oder 2 Nitrogruppen mono-, di- oder trisubstituierten Arylrest mit 6 bis 10 C-Atomen;
bedeuten, durch Mischen mit pharmakologisch annehmbaren Träger- und/oder Zusatzstoffen und gegebenenfalls noch einem oder mehreren anderen therapeutisch wirksamen Stoffen in eine zur Verabreichung geeignete Form gebracht werden.

**Patentansprüche für folgenden Vertragsstaat : GR**

**1.** Substituierte 3-Aminosydnonimine der allgemeinen Formel I

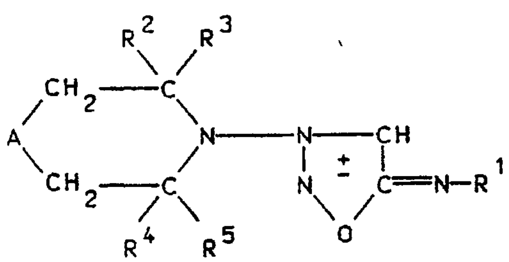

(I)

und ihre pharmakologisch annehmbaren Säureadditionssalze, worin
A den Rest $-CH_2-$, -O-, $-S(O)_n-$, $-N(R^6)-$ oder eine direkte Bindung;
$R^1$ Wasserstoff oder den Rest $-COR^7$;
$R^2$, $R^3$, $R^4$, $R^5$ Alkyl mit 1 bis 4 C-Atomen;
n die Zahl 0, 1 oder 2;
$R^6$ Alkyl mit 1 bis 4 C-Atomen, Hydroxyalkyl mit 1 bis 4 C-Atomen; Phenylalkyl mit 2 bis 4 C-Atomen im Alkylrest;
$R^7$ einen aliphatischen Rest mit 1 bis 4 C-Atomen, der auch durch Alkoxy mit 1 bis 3 C-Atomen substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen; einen Aryloxyrest mit 6 bis 10 C-Atomen; einen Alkoxycarbonylrest mit

insgesamt 2 bis 7 C-Atomen; einen Arylrest mit 6 bis 10 C-Atomen; einen durch 1 bis 3 Halogenatome und/oder 1 bis 3 Alkylreste mit 1 bis 3 C-Atomen und/oder 1 bis 3 Alkoxyreste mit 1 bis 3 C-Atomen und/oder 1 oder 2 Nitrogruppen mono-, di- oder trisubstituierten Arylrest mit 6 bis 10 C-Atomen; bedeuten.

2. Substituierte 3-Aminosydnonimine nach Anspruch 1, dadurch gekennzeichnet, daß $R^1$ Wasserstoff bedeutet.

3. Substituierte 3-Aminosydnonimine nach Anspruch 1 und/oder 2, dadurch gekennzeichnet, daß $R^2$, $R^3$, $R^4$ und $R^5$ Methyl bedeuten.

4. Substituierte 3-Aminosydnonimine nach einem oder mehreren der Ansprüche 1 bis 3, dadurch gekennzeichnet, daß A $-CH_2-$, -O- oder $-N(R^6)-$ bedeutet.

5. 3-(2,2,6,6-Tetramethylpiperidino)-sydnonimin und seine pharmakologisch annehmbaren Säureadditionssalze.

6. 3-(2,2,6,6-Tetramethyl-4-isopropyl-piperazin-1-yl)-sydnonimin und seine pharmakologisch annehmbaren Säureadditionssalze, insbesondere sein Dihydrochlorid.

7. Verfahren zur Herstellung der in einem oder mehreren der Ansprüche 1 bis 4 angegebenen Verbindungen der Formel I oder der in den Ansprüchen 5 und 6 angegebenen Verbindungen, dadurch gekennzeichnet, daß eine Verbindung der allgemeinen Formel II

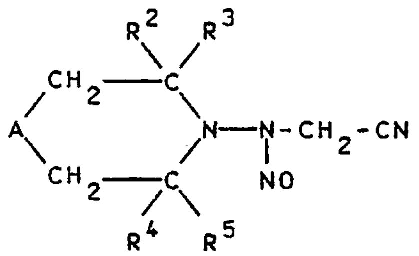

(II)

worin A, $R^2$, $R^3$, $R^4$ und $R^5$ die in einem oder mehreren der Ansprüche 1 bis 5 angegebenen Bedeutungen besitzen, zu einer Verbindung der Formel Ia

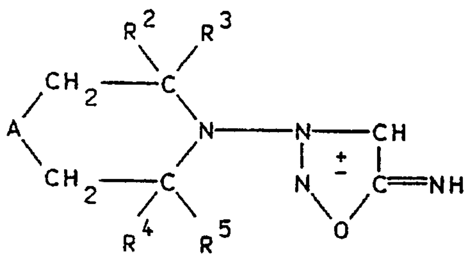

(Ia)

die auch in Form eines Säureadditionssalzes vorliegen kann, cyclisiert wird und gegebenenfalls aus dem Säureadditionssalz die freie Verbindung isoliert wird und daß für den Fall, daß eine Verbindung der Formel I mit $R^1$ = $-COR^7$ hergestellt wird, die Verbindung der Formel Ia oder ein Säureadditionssalz davon mit einem Acylierungsmittel acyliert wird, das den Rest $-COR^7$ einführt und die erhaltene Verbindung gegebenenfalls in ein Säureadditionssalz überführt wird.

8. Verfahren nach Anspruch 7, dadurch gekennzeichnet, daß die Cyclisierung in einem Lösungs- oder Dispergiermittel bei Temperaturen von 0 bis 40 °C, vorzugsweise 0 bis 20 °C, mit Hilfe von Cyclisierungsmitteln, die in wäßriger Lösung einen pH-Wert unter 3 einstellen, durchgeführt wird.

9. Verwendung eines oder mehrerer substituierter 3-Amino-sydnonimine und/oder eines oder mehrerer

ihrer pharmakologisch annehmbaren Säureadditionssalze der Ansprüche 1 bis 6 als pharmakologische Wirkstoffe zusammen mit einem oder mehreren pharmakologisch annehmbaren Träger-und/oder Zusatzstoffen und gegebenenfalls noch einem oder mehreren anderen therapeutsch wirksamen Stoffen zur Herstellung pharmazeutischer Präparate, die zur Anwendung bei der Bekämpfung und Vorbeugung cardiovaskulärer Erkrankungen bestimmt sind.

**10.** Verfahren zur Herstellung eines pharmazeutischen Präparats, dadurch gekennzeichnet, daß ein oder mehrere substituierte 3-Aminosydnonimine der allgemeinen Formel I

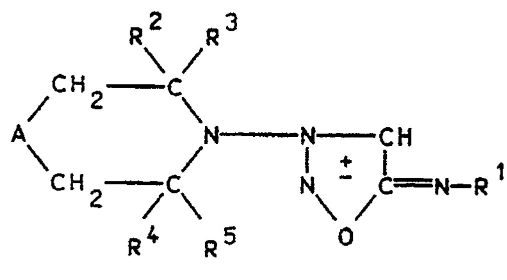

(I)

und/oder ein oder mehrere ihrer pharmakologisch annehmbaren Säureadditionssalze, worin
A den Rest $-CH_2-$, -O-, $-S(O)_n-$, $-N(R^6)-$ oder eine direkte Bindung;
$R^1$ Wasserstoff oder den Rest $-COR^7$;
$R^2$, $R^3$ $R^4$, $R^5$ Alkyl mit 1 bis 4 C-Atomen;
n die Zahl 0, 1 oder 2;
$R^6$ Alkyl mit 1 bis 4 C-Atomen, Hydroxyalkyl mit 1 bis 4 C-Atomen; Phenylalkyl mit 2 bis 4 C-Atomen im Alkylrest;
$R^7$ einen aliphatischen Rest mit 1 bis 4 C-Atomen, der auch durch Alkoxy mit 1 bis 3 C-Atomen substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen durch 1 bis 3 Halogenatome und/oder 1 bis 3 Alkylreste mit 1 bis 3 C-Atomen und/oder 1 bis 3 Alkoxyreste mit 1 bis 3 C-Atomen und/oder 1 oder 2 Nitrogruppen mono-, di- oder trisubstituierten Arylrest mit 6 bis 10 C-Atomen;
bedeuten, durch Mischen mit pharmakologisch annehmbaren Träger- und/oder Zusatzstoffen und gegebenenfalls noch einem oder mehreren anderen therapeutisch wirksamen Stoffen in eine zur Verabreichung geeignete Form gebracht werden.

**Claims**

**Claims for the following Contracting States : DE, FR, GB, CH, IT, BE, NL, AT, SE**

**1.** Substituted 3-aminosydnonimines of the general formula I

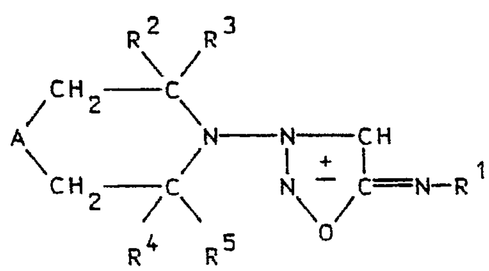

(I)

and their pharmacologically acceptable acid addition salts, wherein
A denotes the radical $-CH_2-$, -O-, $-S(O_2)-$, $-N(R^6)-$ or a direct bond;
$R^1$ denotes hydrogen or the radical $-COR^7$;
$R^2$, $R^3$, $R^4$ and $R^5$ denote alkyl with 1 to 4 C atoms;
n denotes the number 0,1 or 2;
$R^6$ denotes alkyl with 1 to 4 C atoms, hydroxyalkyl with 1 to 4 C atoms; phenylalkyl with 2 to 4 C atoms

in the alkyl radical;
$R^7$ denotes an aliphatic radical which has 1 to 4 C atoms and can also be substituted by alkoxy with 1 to 3 C atoms; a cycloaliphatic radical with 5 to 7 C atoms; a bicycloaliphatic radical with 7 to 14 C atoms; a tricycloaliphatic radical with 7 to 16 C atoms; an alkoxy radical with 1 to 6 C atoms; an aryloxy radical with 6 to 10 C atoms; an alkoxycarbonyl radical with a total of 2 to 7 C atoms; an aryl radical with 6 to 10 C atoms; or an aryl radical which has 6 to 10 C atoms and is mono-, dior trisubstituted by 1 to 3 halogen atoms and/or 1 to 3 alkyl radicals with 1 to 3 C atoms and/or 1 to 3 alkoxy radicals with 1 to 3 C atoms and/or 1 or 2 nitro groups.

2. Substituted 3-aminosydnonimines according to Claim 1, characterized in that $R^1$ denotes hydrogen.

3. Substituted 3-aminosydnonimines according to Claim 1 and/or 2, characterized in that $R^2$, $R^3$, $R^4$ and $R^5$ all denote methyl.

4. Substituted 3-aminosydnonimines according to one or more of Claims 1 to 3, characterized in that A denotes $-CH_2-$, -O- or $-N(R^6)-$.

5. 3-(2,2,6,6-Tetramethylpiperidino)-sydnonimine and its pharmacologically acceptable acid addition salts.

6. 3-(2,2,6,6-Tetramethyl-4-isopropyl-piperazin-1-yl)-sydnonimine and its pharmacologically acceptable acid addition salts, in particular its dihydrochloride.

7. Process for the preparation of the compounds of the formula I described in one or more of Claims 1 to 4 or of the compounds described in Claims 5 and 6, characterized in that a compound of the general formula II

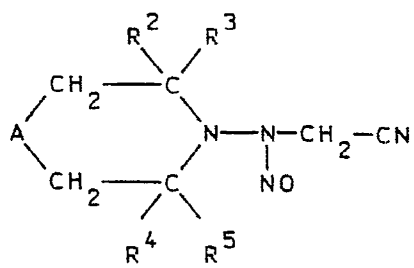

(II)

wherein A, $R^2$, $R^3$, $R^4$ and $R^5$ have the meanings given in one or more of Claims 1 to 5, is cyclized to a compound of the formula Ia

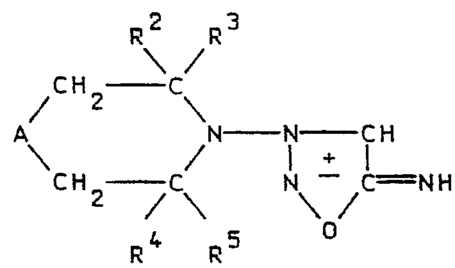

(Ia)

which can also be in the form of an acid addition salt, and, if appropriate, the free compound is isolated from the acid addition salt, and in that in the case where a compound of the formula I where $R^1$ = $-COR^7$ is prepared, the compound of the formula Ia or an acid addition salt thereof is acylated with an acylating agent which introduces the radical $-COR^7$, and if appropriate the resulting compound is converted into an acid addition salt.

8. Process according to Claim 7, characterized in that the cyclization is carried out in a solvent or dispersing agent at temperatures of 0 to 40 °C, preferably 0 to 20 °C, with the aid of cyclizing agents which establish a pH of less than 3 in aqueous solution.

9. Use of one or more substituted 3-aminosydnonimines and/or one or more of pharmacologically acceptable acid addition salts thereof of Claims 1 to 6 as pharmacological active compounds together with one or more pharmacologically acceptable excipients and/or additives and, if appropriate, one or more other therapeutically active substances for preparing pharmaceutical preparations for use in combating and preventing cardiovascular diseases.

10. Pharmaceutical preparation, characterized in that it contains one or more compounds of Claims 1 to 6 or one or more acid addition salts thereof as active compounds, together with pharmaceutically acceptable excipients and/or additives and if appropriate also one or more other therapeutically active compounds.

**Claims for the following Contracting State : ES**

1. Process for preparing substituted 3-aminosydnonimines of the general formula I

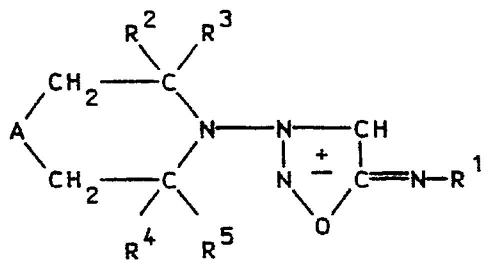

(I)

and their pharmacologically acceptable acid addition salts, wherein
A denotes the radical $-CH_2-$, -O-, $-S(O_2)-$, $-N(R^6)-$ or a direct bond;
$R^1$ denotes hydrogen or the radical $-COR^7$;
$R^2$, $R^3$, $R^4$ and $R^5$ denote alkyl with 1 to 4 C atoms;
n denotes the number 0,1 or 2;
$R^6$ denotes alkyl with 1 to 4 C atoms, hydroxyalkyl with 1 to 4 C atoms; phenylalkyl with 2 to 4 C atoms in the alkyl radical;
$R^7$ denotes an aliphatic radical which has 1 to 4 C atoms and can also be substituted by alkoxy with 1 to 3 C atoms; a cycloaliphatic radical with 5 to 7 C atoms; a bicycloaliphatic radical with 7 to 14 C atoms; a tricycloaliphatic radical with 7 to 16 C atoms; an alkoxy radical with 1 to 6 C atoms; an aryloxy radical with 6 to 10 C atoms; an alkoxycarbonyl radical with a total of 2 to 7 C atoms; an aryl radical with 6 to 10 C atoms; or an aryl radical which has 6 to 10 C atoms and is mono-, dior trisubstituted by 1 to 3 halogen atoms and/or 1 to 3 alkyl radicals with 1 to 3 C atoms and/or 1 to 3 alkoxy radicals with 1 to 3 C atoms and/or 1 or 2 nitro groups; characterized in that a compound of the general formula II

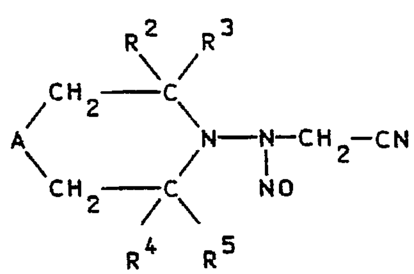

(II)

wherein A, $R^2$, $R^3$, $R^4$ and $R^5$ have the meanings given in one or more of Claims 1 to 5, is cyclized to a compound of the formula Ia

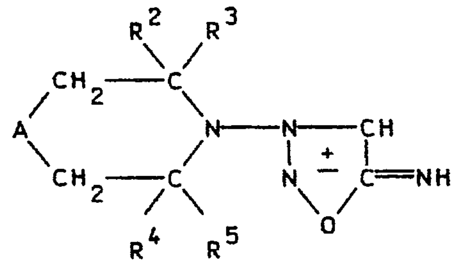

(Ia)

which can also be in the form of an acid addition salt, and, if appropriate, the free compound is isolated from the acid addition salt, and in that in the case where a compound of the formula I where $R^1$ = $COR^7$ is prepared, the compound of the formula Ia or an acid addition salt thereof is acylated with an acylating agent which introduces the radical $-COR^7$, and if appropriate the resulting compound is converted into an acid addition salt.

2. Process according to Claim 1, characterized in that the compound Ia is isolated without acylation or is converted into an acid addition salt without acylation.

3. Process according to Claim 1 and/or 2, characterized in that a compound of formula II is employed wherein $R^2$, $R^3$, $R^4$ and $R^5$ denote methyl.

4. Process according to one or more of Claims 1 to 3, characterized in that a compound of formula II is employed wherein A denotes $-CH_2-$.

5. Process according to one or more of Claims 1 to 3, characterized in that a compound of formula II is employed wherein A denotes -O-.

6. Process according to one or more of Claims 1 to 3, characterized in that a compound of formula II is employed wherein A denotes $-N(R^6)-$.

7. Process according to Claim 1, characterized in that a compound of formula II is employed wherein $R^2$, $R^3$, $R^4$ and $R^5$ denote methyl and A denotes $-N(-CH(CH_3)_2)$ and that the compound Ia is isolated without acylation or is converted into an acid addition dalt, in particular into the dihydrochloride, without acylation.

8. Process according to Claim 1, characterized in that a compound of formula II is employed wherein $R^2$, $R^3$, $R^4$ and $R^5$ denote methyl and A denotes $-CH_2-$ and that the compound Ia is isolated without acylation or is converted into an acid addition salt without acylation.

9. Use or one or more substituted 3-aminosydnonimines of the general formula I

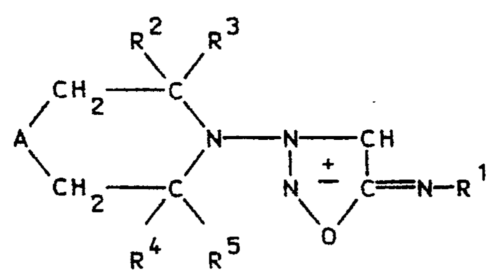

(I)

and/or one or more of their pharmacologically acceptable acid addition salts, wherein
A denotes the radical $-CH_2-$, -O-, $-S(O_2)-$, $-N(R^6)-$ or a direct bond;
$R^1$ denotes hydrogen or the radical $-COR^7$;
$R^2$, $R^3$, $R^4$ and $R^5$ denote alkyl with 1 to 4 C atoms;
n denotes the number 0,1 or 2;
$R^6$ denotes alkyl with 1 to 4 C atoms, hydroxyalkyl with 1 to 4 C atoms; phenylalkyl with 2 to 4 C atoms

in the alkyl radical;
$R^7$ denotes an aliphatic radical which has 1 to 4 C atoms and can also be substituted by alkoxy with 1 to 3 C atoms; a cycloaliphatic radical with 5 to 7 C atoms; a bicycloaliphatic radical with 7 to 14 C atoms; a tricycloaliphatic radical with 7 to 16 C atoms; an alkoxy radical with 1 to 6 C atoms; an aryloxy radical with 6 to 10 C atoms; an alkoxycarbonyl radical with a total of 2 to 7 C atoms; an aryl radical with 6 to 10 C atoms; or an aryl radical which has 6 to 10 C atoms and is mono-, di-or trisubstituted by 1 to 3 halogen atoms and/or 1 to 3 alkyl radicals with 1 to 3 C atoms and/or 1 to 3 alkoxy radicals with 1 to 3 C atoms and/or 1 or 2 nitro groups, together with pharmacologically acceptable excipients and/or additives and, if appropriate, one or more other therapeutically active substances for preparing pharmaceutical preparations for use in combating and preventing cardiovascular diseases.

**10.** Process for preparing a pharmaceutical preparation, characterized in that one or more substituted 3-aminosydnonimines of the general formula I

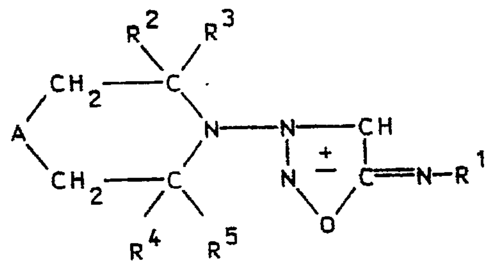

(I)

and/or one or more of their pharmacologically acceptable acid addition salts, wherein
A denotes the radical $-CH_2-$, -O-, $-S(O_2)-$, $-N(R^6)-$ or a direct bond;
$R^1$ denotes hydrogen or the radical $-COR^7$;
$R^2$, $R^3$, $R^4$ and $R^5$ denote alkyl with 1 to 4 C atoms;
n denotes the number 0,1 or 2;
$R^6$ denotes alkyl with 1 to 4 C atoms, hydroxyalkyl with 1 to 4 C atoms; phenylalkyl with 2 to 4 C atoms in the alkyl radical;
$R^7$ denotes an aliphatic radical which has 1 to 4 C atoms and can also be substituted by alkoxy with 1 to 3 C atoms; a cycloaliphatic radical with 5 to 7 C atoms; a bicycloaliphatic radical with 7 to 14 C atoms; a tricycloaliphatic radical with 7 to 16 C atoms; an alkoxy radical with 1 to 6 C atoms; an aryloxy radical with 6 to 10 C atoms; an alkoxycarbonyl radical with a total of 2 to 7 C atoms; an aryl radical with 6 to 10 C atoms; or an aryl radical which has 6 to 10 C atoms and is mono-, di-or trisubstituted by 1 to 3 halogen atoms and/or 1 to 3 alkyl radicals with 1 to 3 C atoms and/or 1 to 3 alkoxy radicals with 1 to 3 C atoms and/or 1 or 2 nitro groups, are converted into a form suitable for administration by mixing with pharmacologically acceptable excipients and/or additives and, if appropriate, one ore more other therapeutically active substances.

**Claims for the following Contracting State : GR**

**1.** Substituted 3-aminosydnonimines of the general formula I

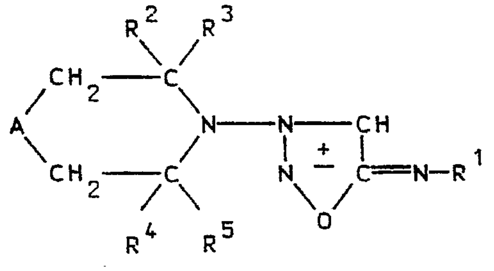

(I)

and their pharmacologically acceptable acid addition salts, wherein
A denotes the radical $-CH_2-$, -O-, $-S(O_2)-$, $-N(R^6)-$ or a direct bond;
$R^1$ denotes hydrogen or the radical $-COR^7$;

$R^2$, $R^3$, $R^4$ and $R^5$ denote alkyl with 1 to 4 C atoms;
n denotes the number 0,1 or 2;
$R^6$ denotes alkyl with 1 to 4 C atoms, hydroxyalkyl with 1 to 4 C atoms; phenylalkyl with 2 to 4 C atoms in the alkyl radical;
$R^7$ denotes an aliphatic radical which has 1 to 4 C atoms and can also be substituted by alkoxy with 1 to 3 C atoms; a cycloaliphatic radical with 5 to 7 C atoms; a bicycloaliphatic radical with 7 to 14 C atoms; a tricycloaliphatic radical with 7 to 16 C atoms; an alkoxy radical with 1 to 6 C atoms; an aryloxy radical with 6 to 10 C atoms; an alkoxycarbonyl radical with a total of 2 to 7 C atoms; an aryl radical with 6 to 10 C atoms; or an aryl radical which has 6 to 10 C atoms and is mono-, di-or trisubstituted by 1 to 3 halogen atoms and/or 1 to 3 alkyl radicals with 1 to 3 C atoms and/or 1 to 3 alkoxy radicals with 1 to 3 C atoms and/or 1 or 2 nitro groups.

2. Substituted 3-aminosydnonimines according to Claim 1, characterized in that $R^1$ denotes hydrogen.

3. Substituted 3-aminosydnonimines according to Claim 1 and/or 2, characterized in that $R^2$, $R^3$, $R^4$, and $R^5$ all denote methyl.

4. Substituted 3-aminosydnonimines according to one or more of Claims 1 to 3, characterized in that A denotes $-CH_2-$, -O- or $-N(R^6)-$.

5. 3-(2,2,6,6-Tetramethylpiperidino)-sydnonimine and its pharmacologically acceptable acid addition salts.

6. 3-(2,2,6,6-Tetramethyl-4-isopropyl-piperazin-1-yl)-sydnonimine and its pharmacologically acceptable acid addition salts, in particular its dihydrochloride.

7. Process for the preparation of the compounds of the formula I described in one or more of Claims 1 to 4 or of the compounds described in Claims 5 and 6, characterized in that a compound of the general formula II

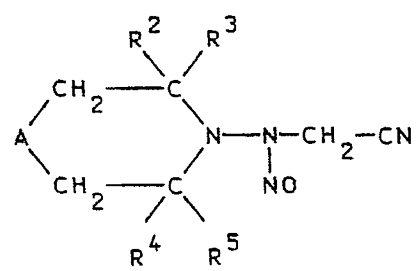

(II)

wherein A, $R^2$, $R^3$, $R^4$ and $R^5$ have the meanings given in one or more of Claims 1 to 5, is cyclized to a compound of the formula Ia

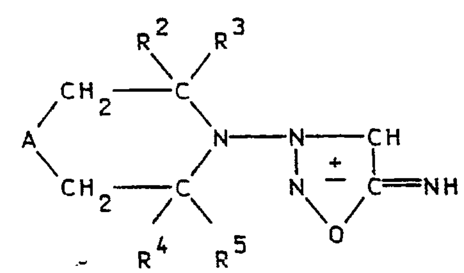

(Ia)

which can also be in the form of an acid addition salt, and, if appropriate, the free compound is isolated from the acid addition salt, and in that in the case where a compound of the formula I where $R^1$ = $-COR^7$ is prepared, the compound of the formula Ia or an acid addition salt thereof is acylated with an acylating agent which introduces the radical $-COR^7$, and if appropriate the resulting compound is converted into an acid addition salt.

**8.** Process according to Claim 7, characterized in that the cyclization is carried out in a solvent or dispersing agent at temperatures of 0 to 40° C, preferably 0 to 20° C, with the aid of cyclizing agents which establish a pH of less than 3 in aqueous solution.

**9.** Use of one or more substituted 3-aminosydnonimines and/or one or more of pharmacologically acceptable acid addition salts thereof of Claims 1 to 6 as pharmacological active compounds together with one or more pharmacologically acceptable excipients and/or additives and, if appropriate, one or more other therapeutically active substances for preparing pharmaceutical preparations for use in combating and preventing cardiovascular diseases.

**10.** Process for preparing a pharmaceutical preparation, characterized in that one or more substituted 3-aminosydnonimines of the general formula I

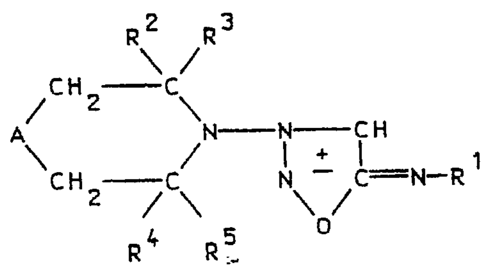

(I)

and/or one or more of their pharmacologically acceptable acid addition salts, wherein
A denotes the radical $-CH_2-$, -O-, $-S(O_2)-$, $-N(R^6)-$ or a direct bond;
$R^1$ denotes hydrogen or the radical $-COR^7$;
$R^2$, $R^3$, $R^4$ and $R^5$ denote alkyl with 1 to 4 C atoms;
n denotes the number 0,1 or 2;
$R^6$ denotes alkyl with 1 to 4 C atoms, hydroxyalkyl with 1 to 4 C atoms; phenylalkyl with 2 to 4 C atoms in the alkyl radical;
$R^7$ denotes an aliphatic radical which has 1 to 4 C atoms and can also be substituted by alkoxy with 1 to 3 C atoms; a cycloaliphatic radical with 5 to 7 C atoms; a bicycloaliphatic radical with 7 to 14 C atoms; a tricycloaliphatic radical with 7 to 16 C atoms; an alkoxy radical with 1 to 6 C atoms; an aryloxy radical with 6 to 10 C atoms; an alkoxycarbonyl radical with a total of 2 to 7 C atoms; an aryl radical with 6 to 10 C atoms; or an aryl radical which has 6 to 10 C atoms and is mono-, di-or trisubstituted by 1 to 3 halogen atoms and/or 1 to 3 alkyl radicals with 1 to 3 C atoms and/or 1 to 3 alkoxy radicals with 1 to 3 C atoms and/or 1 or 2 nitro groups, are converted into a form suitable for administration by mixing with pharmacologically acceptable excipients and/or additives and, if appropriate, one ore more other therapeutically active substances.

**Revendications**

**Revendications pour les Etats contractants suivants : DE, FR, GB, CH, IT, BE, NL, AT, SE**

**1.** 3-aminosydnonimines substituées répondant à la formule générale I

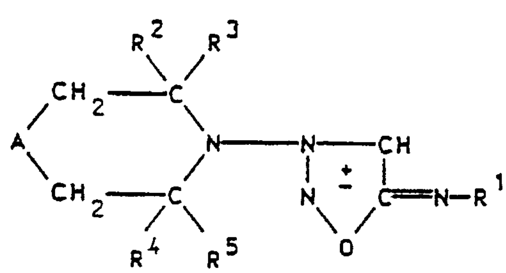

(I)

et leurs sels d'addition aux acides pharmaceutiquement acceptables, dans laquelle
A désigne les radicaux $-CH_2-$, -O-, $-S(O)_n-$, $-N(R^6)-$ ou une liaison directe
$R^1$ désigne un atome d'hydrogène ou le radical $-COR^7$ ;

$R^2$, $R^3$, $R^4$, $R^5$ désignent un groupe alkyle en $C_1$-$C_4$ ;
n est 0, 1 ou 2 ;
$R^6$ désigne un groupe alkyle en $C_1$-$C_4$ ; hydroxyalkyle en $C_1$-$C_4$ ; un groupe phénylalkyle ayant 2 à 4 atomes de carbone dans le radical alkyle ;
$R^7$ désigne un radical aliphatique en $C_1$-$C_4$, qui peut aussi être susbstitué par un groupe alcoxy en $C_1$-$C_3$ ; un radical cycloaliphatique en $C_1$-$C_7$ ; un radical bicycloaliphatique en $C_7$-$C_{14}$ ; un radical tricycloaliphatique en $C_7$-$C_{16}$ ; un radical alcoxy en $C_1$-$C_6$ ; un radical aryloxy en $C_6$-$C_{10}$ ; un radical alcoxycarbonyle ayant au total 2 à 7 atomes de carbone ; un radical aryle en $C_6$-$C_{10}$ ; un radical aryle en $C_6$-$C_{10}$ mono-, diou trisubstitué par 1 à 3 atomes d'halogène et/ou 1 à 3 radicaux alkyles en $C_1$-$C_3$ et/ou 1 à 3 radicaux alcoxys en $C_1$-$C_3$ et/ou 1 ou 2 groupes nitros ;
n est égal à 0, 1 ou 2.

2. 3-aminosydnonimines substituées selon la revendication 1, caractérisées en ce que $R^1$ désigne un atome d'hydrogène.

3. 3-aminosydnonimines substituées selon les revendications 1 et/ou 2, caractérisées en ce que $R^2$, $R^3$, $R^4$, $R^5$ désignent des groupes méthyles.

4. 3-aminosydnonimines substituées selon une ou plusieurs des revendications 1 à 3, caractérisées en ce que A désigne les radicaux $-CH_2-$, -O- ou $-N(R^6)-$.

5. 3-(2,2,6,6-tétraméthylpipéridino)-sydnonimine et ses sels d'addition aux acides pharmacologiquement acceptables.

6. 3-(2,2,6,6-tétraméthyl-4-isopropyl-pipérazin-1-yl)-sydnonimine et ses sels d'addition aux acides pharmacologiquement acceptables, en particulier son dichlorhydrate.

7. Procédé de préparation des composés répondant à la formule I indiqués dans une ou plusieurs des revendications 1 à 4 ou des composés indiqués dans les revendications 5 et 6, caractérisé en ce qu'on cyclise un composé répondant à la formule générale II

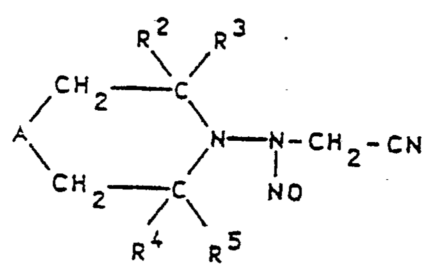
(II)

dans laquelle A, $R^2$, $R^3$, $R^4$ et $R^5$ possèdent les significations indiquées dans une ou plusieurs des revendications 1 à 5, pour donner un composé répondant à la formule générale Ia

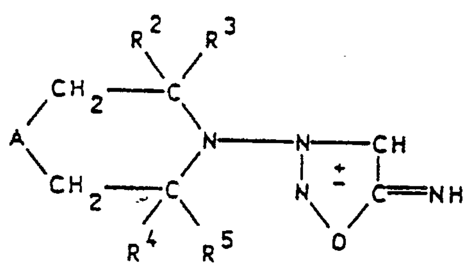
(Ia)

qui peut également se présenter sous la forme d'un sel d'addition aux acides, et en ce qu'on isole le cas échéant le composé libre du sel d'addition aux acides et en ce que, dans le cas où on prépare un composé répondant à la formule I avec $R^1$ = $-COR^7$, on acyle le composé répondant à la formule Ia ou un de ses sels d'addition aux acides avec un agent d'acylation qui introduit le radical $-COR^7$ et on

transforme le cas échéant le composé obtenu en un sel d'addition aux acides.

**8.** Procédé selon la revendication 7, caractérisé en ce que la cyclisation est effectuée dans un solvant ou un dispersant, à des températures de 0 à 40 °C, de préférence de 0 à 20 °C, à l'aide d'agents de cyclisation qui ajustent en solution aqueuse un pH inférieur à 3.

**9.** Utilisation d'une ou plusieurs 3-amino-sydnonimines substituées et/ou d'un ou plusieurs de leurs sels d'addition aux acides pharmacologiquement acceptables selon les revendications 1 à 6 comme principes actifs pharmacologiques en même temps qu'un ou plusieurs excipients et/ou additifs pharmacologiquement acceptables et le cas échéant en outre une ou plusieurs autres substances thérapeutiquement actives pour la confection de préparations pharmaceutiques qui sont destinées à la lutte contre les maladies cardiovasculaires et à leur prévention.

**10.** Préparation pharmaceutique, caractérisée en ce qu'elle contient un ou plusieurs composés selon les revendications 1 à 6 ou un ou plusieurs sels d'addition aux acides de ceux-ci comme principes actifs, avec des excipients et/ou additifs pharmacologiquement acceptables et le cas échéant en outre une ou plusieurs autres substances thérapeutiquement actives.

**Revendications pour l'Etat contractant suivant : ES**

**1.** Procédé de préparation de 3-aminosydnonimines substituées répondant à la formule générale I

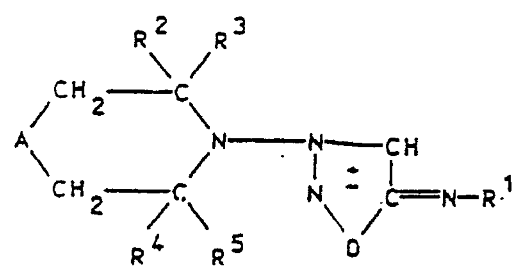

(I)

et de leurs sels d'addition aux acides pharmacologiquement acceptables, dans laquelle
A désigne les radicaux $-CH_2-$, -O-, $-S(O)_n-$, $-N(R^6)-$ ou une liaison directe
$R^1$ désigne l'hydrogène ou le radical $-COR^7$ ;
$R^2$, $R^3$, $R^4$, $R^5$ désignent des groupes alkyles en $C_1$-$C_4$ ;
n est 0, 1 ou 2 ;
$R^6$ désigne un groupe alkyle en $C_1$-$C_4$ ; hydroxyalkyle en $C_1$-$C_4$ ; un groupe phénylalkyle ayant 2 à 4 atomes de carbone dans le groupe alkyle ;
$R^7$ désigne un radical aliphatique en $C_1$-$C_4$ qui peut aussi être susbstitué par un groupe alcoxy en $C_1$-$C_3$ ; un radical cycloaliphatique en $C_1$-$C_7$ ; un radical bicycloaliphatique en $C_7$-$C_{14}$ ; un radical tricycloaliphatique en $C_7$-$C_{16}$ ; un radical alcoxy en $C_1$-$C_6$ ; un radical aryloxy en $C_6$-$C_{10}$ ; un radical alcoxycarbonyle ayant au total 2 à 7 atomes de carbone ; un radical aryle en $C_6$-$C_{10}$ ; un radical aryle en $C_6$-$C_{10}$ mono-, di-ou trisubstitué par 1 à 3 atomes d'halogène et/ou 1 à 3 groupes alkyles en $C_1$-$C_3$ et/ou 1 à 3 groupes alcoxys en $C_1$-$C_3$ et/ou 1 ou 2 groupes nitros ;
n est égal à 0, 1 ou 2,
caractérisé en ce qu'on cyclise un composé répondant à la formule générale II

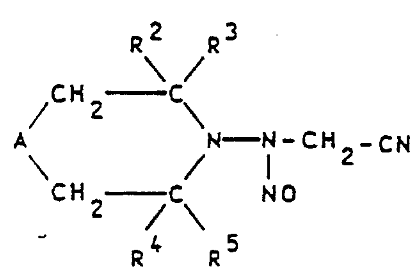

(II)

dans laquelle A, $R^2$, $R^3$, $R^4$ et $R^5$ possèdent les significations indiquées dans une ou plusieurs des revendications 1 à 5, pour donner un composé répondant à la formule Ia

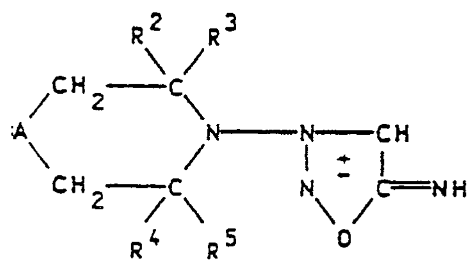

(Ia)

qui peut également se présenter sous la forme d'un sel d'addition aux acides, et qu'on isole le cas échéant le composé libre du sel d'addition aux acides et que, dans le cas où on prépare un composé répondant à la formule I avec $R^1$ = $-COR^7$, on acyle le composé répondant à la formule Ia ou un de ses sels d'addition aux acides avec un agent d'acylation qui introduit le radical $-COR^7$ et on transforme le cas échéant le composé obtenu en un sel d'addition aux acides.

2. Procédé selon la revendication 1, caractérisé en ce qu'on isole le composé Ia sans acylation, ou on le transforme sans acylation en un sel d'addition aux acides.

3. Procédé selon les revendications 1 et/ou 2, caractérisé en ce qu'on utilise un composé répondant à la formule II dans laquelle $R^2$, $R^3$, $R^4$, $R^5$ désignent des groupes méthyles.

4. Procédé selon une ou plusieurs des revendications 1 à 3, caractérisé en ce qu'on utilise un composé répondant à la formule II dans laquelle A désigne $-CH_2-$.

5. Procédé selon une ou plusieurs des revendications 1 à 3, caractérisé en ce qu'on utilise un composé répondant à la formule II dans laquelle A désigne -O-.

6. Procédé selon une ou plusieurs des revendications 1 à 3, caractérisé en ce qu'on utilise un composé répondant à la formule II dans laquelle A désigne $-N(R^6)-$.

7. Procédé selon la revendication 1, caractérisé en ce qu'on utilise un composé répondant à la formule générale II, dans laquelle $R^2$, $R^3$, $R^4$ et $R^5$ désignent des groupes méthyles et A un groupe $-N(-CH(CH_3)_2)-$ et en ce qu'on isole le composé Ia sans acylation ou on le transforme sans acylation en un sel d'addition aux acides, en particulier le dichlorhydrate.

8. Procédé selon la revendication 1, caractérisé en ce qu'on utilise un composé répondant à la formule générale II, dans laquelle $R^2$, $R^3$, $R^4$ et $R^5$ désignent des groupes méthyles et A un groupe $-CH_2-$, et en ce qu'on isole le composé Ia sans acylation ou en ce qu'on le transforme sans acylation en un sel d'addition aux acides.

9. Utilisation d'une ou plusieurs 3-aminosydnonimines substituées répondant à la formule générale I

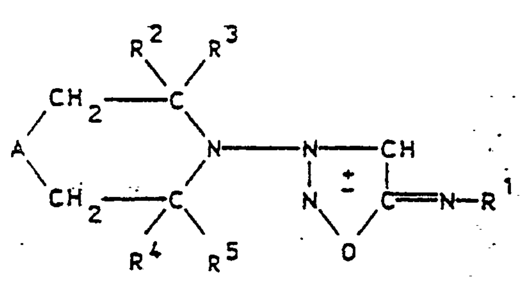

(I)

et/ou un ou plusieurs de leurs sels d'addition aux acides pharmacologiquement acceptables, dans

laquelle
A désigne les radicaux $-CH_2-$, -O-, $-S(O)_n-$, $-N(R^6)-$ ou une liaison directe ;
$R^1$ désigne l'hydrogène ou le radical $-COR^7$ ;
$R^2$, $R^3$, $R^4$, $R^5$ désignent des groupes alkyles en $C_1$-$C_4$ ;
n les nombres 0, 1 ou 2 ;
$R^6$ désigne un groupe alkyle en $C_1$-$C_4$ ; hydroxyalkyle en $C_1$-$C_4$ ; un groupe phénylalkyle ayant 2 à 4 atomes de carbone dans le radical alkyle ;
$R^7$ désigne un radical aliphatique en $C_1$-$C_4$, qui peut aussi être substitué par un groupe alcoxy en $C_1$-$C_3$ ; un radical cycloaliphatique en $C_1$-$C_7$ ; un radical bicycloaliphatique en $C_7$-$C_{14}$ ; un radical tricycloaliphatique en $C_7$-$C_{16}$ ; un radical alcoxy en $C_1$-$C_6$ ; un radical aryloxy en $C_6$-$C_{10}$ ; un radical alcoxycarbonyle ayant au total 2 à 7 atomes de carbone ; un radical alkyle en $C_6$-$C_{10}$ ; un radical aryle en $C_6$-$C_{10}$ mono-, di- ou trisubstitué par 1 à 3 atomes d'halogène et/ou 1 à 3 radicaux alkyles en $C_1$-$C_3$ et/ou 1 à 3 radicaux alcoxys en $C_1$-$C_3$ et/ou 1 ou 2 groupes nitros ;
n les nombres 0, 1 ou 2,
en même temps que des excipients et/ou additifs pharmacologiquement acceptables, et le cas échéant en outre une ou plusieurs autres substances thérapeutiquement actives pour la confection de préparation pharmaceutiques qui sont destinées à l'utilisation pour la lutte contre les maladies cardiovasculaires et leur prévention.

**10.** Procédé de confection d'une préparation pharmaceutique, caractérisé en ce qu'on amène sous une forme d'administration appropriée une ou plusieurs 3-aminosydnonimines substituées répondant à la formule générale I

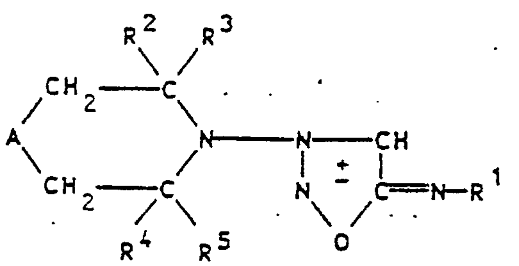

(I)

et/ou un ou plusieurs de leurs sels d'addition aux acides pharmacologiquement acceptables, dans laquelle
A désigne les radicaux $-CH_2-$, -O-, $-S(O)_n-$, $-N(R^6)-$ ou une liaison directe ;
$R^1$ désigne l'atome d'hydrogène ou le radical $-COR^7$ ;
$R^2$, $R^3$, $R^4$, $R^5$ désignent des groupes alkyles en $C_1$-$C_4$ ;
n les nombres 0, 1 ou 2 ;
$R^6$ désigne un groupe alkyle en $C_1$-$C_4$ ; hydroxyalkyle en $C_1$-$C_4$ ; un groupe phénylalkyle ayant 2 à 4 atomes de carbone dans le radical alkyle ;
$R^7$ désigne un radical aliphatique en $C_1$-$C_4$, qui peut aussi être susbstitué par un groupe alcoxy en $C_1$-$C_3$ ; un radical cycloaliphatique en $C_5$-$C_7$ ; un radical bicycloaliphatique en $C_7$-$C_{14}$ ; un radical tricycloaliphatique en $C_7$-$C_{16}$ ; un radical alcoxy en $C_1$-$C_6$ ; un radical aryloxy en $C_6$-$C_{10}$ ; un radical alcoxycarbonyle ayant au total 2 à 7 atomes de carbone ; un radical aryle en $C_6$-$C_{10}$ ; un radical aryle en $C_6$-$C_{10}$ mono-, di-ou trisubstitué par 1 à 3 atomes d'halogène et/ou 1 à 3 groupes alkyles en $C_1$-$C_3$ et/ou 1 à 3 groupes alcoxys en $C_1$-$C_3$ et/ou 1 ou 2 groupes nitros ;
n les nombres 0, 1 ou 2,
par mélange avec des excipients et/ou additifs pharmacologiquement acceptables, et le cas échéant en outre une ou plusieurs autres substances thérapeutiquement actives.

**Revendications pour l'Etat contractant suivant : GR**

**1.** 3-aminosydnonimines substituées répondant à la formule générale I

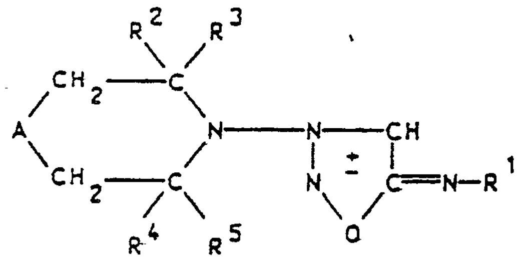

(I)

et leurs sels d'addition aux acides pharmaceutiquement acceptables, dans laquelle
A désigne les radicaux $-CH_2-$, -O-, $-S(O)_n-$, $-N(R^6)-$ ou une liaison directe
$R^1$ désigne un atome d'hydrogène ou le radical $-COR^7$ ;
$R^2$, $R^3$, $R^4$, $R^5$ désignent des groupes alkyles en $C_1$-$C_4$ ;
n les nombres 0, 1 ou 2 ;
$R^6$ un groupe alkyle en $C_1$-$C_4$ ; hydroxyalkyle en $C_1$-$C_4$ ; un groupe phénylalkyle ayant 2 à 4 atomes de carbone dans le radical alkyle ;
$R^7$ un radical aliphatique en $C_1$-$C_4$, qui peut aussi être susbstitué par un groupe alcoxy en $C_1$-$C_3$ ; un radical cycloaliphatique en $C_5$-$C_7$ ; un radical bicycloaliphatique en $C_7$-$C_{14}$ ; un radical tricycloaliphatique en $C_7$-$C_{16}$ ; un radical alcoxy en $C_1$-$C_6$ ; un radical aryloxy en $C_6$-$C_{10}$ ; un radical alcoxycarbonyle ayant au total 2 à 7 atomes de carbone ; un radical aryle en $C_6$-$C_{10}$ ; un radical aryle en $C_6$-$C_{10}$ mono-, di-ou trisubstitué par 1 à 3 atomes d'halogène et/ou 1 à 3 groupes alkyles en $C_1$-$C_3$ et/ou 1 à 3 groupes alcoxys en $C_1$-$C_3$ et/ou 1 ou 2 groupes nitros ;
n est 0, 1 ou 2.

2. 3-aminosydnonimines substituées selon la revendication 1, caractérisées en ce que $R^1$ désigne un atome d'hydrogène.

3. 3-aminosydnonimines substituées selon les revendications 1 et/ou 2, caractérisées en ce que $R^2$, $R^3$, $R^4$, $R^5$ désignent des radicaux méthyles.

4. 3-aminosydnonimines substituées selon une ou plusieurs des revendications 1 à 3, caractérisées en ce que A désigne un radical $-CH_2-$, -O- ou $-N(R^6)-$.

5. 3-(2,2,6,6-tétraméthylpipéridino)-sydnonimine et ses sels d'addition aux acides pharmacologiquement acceptables.

6. 3-(2,2,6,6-tétraméthyl-4-isopropyl-pipérazin-1-yl)-sydnonimine et ses sels d'addition aux acides pharmacologiquement acceptables, en particulier son dichlorhydrate.

7. Procédé de préparation des composés répondant à la formule I indiqués dans une ou plusieurs des revendications 1 à 4 et/ou des composés indiqués dans les revendications 5 et 6, caractérisé en ce qu'on cyclise un composé répondant à la formule générale II

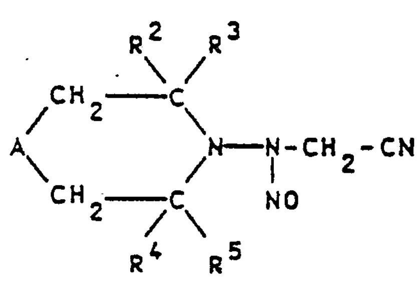

(II)

dans laquelle A, $R^2$, $R^3$, $R^4$ et $R^5$ possèdent les significations indiquées dans une ou plusieurs des revendications 1 à 5, pour donner un composé répondant à la formule générale Ia

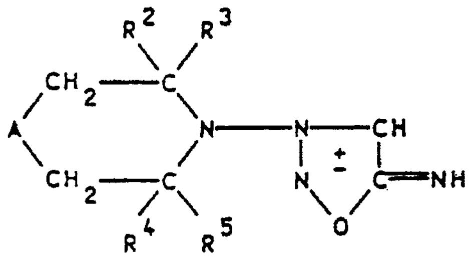

(Ia)

qui peut également se présenter sous la forme d'un sel d'addition aux acides, et en ce qu'on isole le cas échéant le composé libre du sel d'addition aux acides, en ce que, dans le cas où l'on prépare un composé répondant à la formule I avec $R^1$ = $-COR^7$, on acyle le composé répondant à la formule Ia ou un de ses sels d'addition aux acides avec un agent d'acylation qui introduit le radical $-COR^7$, et en ce qu'on transforme le cas échéant le composé obtenu en un sel d'addition aux acides.

**8.** Procédé selon la revendication 7, caractérisé en ce que la cyclisation est effectuée dans un solvant ou un dispersant, à des températures de 0 à 40 °C, de préférence de 0 à 20 °C, à l'aide d'agents de cyclisation qui ajustent en solution aqueuse un pH inférieur à 3.

**9.** Utilisation d'une ou plusieurs 3-amino-sydnonimines substituées et/ou d'un ou plusieurs de leurs sels d'addition aux acides pharmacologiquement acceptables des revendications 1 à 6 comme principes actifs pharmacologiques en même temps qu'un ou plusieurs excipients et/ou additifs pharmacologiquement acceptables, et le cas échéant en outre une ou plusieurs autres substances thérapeutiquement actives pour la confection de préparations pharmaceutiques qui sont destinées à l'utilisation dans la lutte contre les maladies cardiovasculaires et dans leur prévention.

**10.** Procédé de confection d'une préparation pharmaceutique, caractérisé en ce qu'on amène sous une forme d'administration appropriée une ou plusieurs 3-aminosydnonimines substituées répondant à la formule générale I

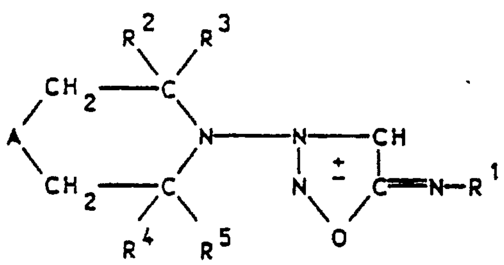

(I)

et/ou un ou plusieurs de leurs sels d'addition aux acides pharmacologiquement acceptables, dans laquelle

A désigne les radicaux $-CH_2-$, $-O-$, $-S(O)_n-$, $-N(R^6)-$ ou une liaison directe ;

$R^1$ désigne un atome d'hydrogène ou le radical $-COR^7$ ;

$R^2$, $R^3$, $R^4$, $R^5$ désignent des groupes alkyles en $C_1$-$C_4$ ;

n est 0, 1 ou 2 ;

$R^6$ désigne un groupe alkyle en $C_1$-$C_4$ ; hydroxyalkyle en $C_1$-$C_4$ ; un groupe phénylalkyle ayant 2 à 4 atomes de carbone dans le radical alkyle ;

$R^7$ désigne un radical aliphatique en $C_1$-$C_4$, qui peut aussi être susbstitué par un groupe alcoxy en $C_1$-$C_3$ ; un radical cycloaliphatique en $C_5$-$C_7$ ; un radical bicycloaliphatique en $C_7$-$C_{14}$ ; un radical tricycloaliphatique en $C_7$-$C_{16}$ ; un radical alcoxy en $C_1$-$C_6$ ; un radical aryloxy en $C_6$-$C_{10}$ ; un radical alcoxycarbonyle ayant au total 2 à 7 atomes de carbone ; un radical aryle en $C_6$-$C_{10}$ ; un radical aryle en $C_6$-$C_{10}$ mono-, di-ou trisubstitué par 1 à 3 atomes d'halogène et/ou 1 à 3 groupes alkyles en $C_1$-$C_3$ et/ou 1 à 3 groupes alcoxys en $C_1$-$C_3$ et/ou 1 ou 2 groupes nitros ;

n est 0, 1 ou 2,

par mélange avec des excipients et/ou des additifs pharmacologiquement acceptables et le cas échéant en outre avec une ou plusieurs autres substances thérapeutiquement actives.